# EUROPEAN PATENT SPECIFICATION

(11) **EP 4 279 923 B1**
(45) Date of publication and mention of the grant of the patent: **10.09.2025**
(21) Application number: 22739499.6
(22) Date of filing: 14.01.2022
(51) Int. Cl.: G01N 33/569, C07K 16/12, C12N 15/13, C12Q 1/04, G01N 33/543

(54) **METHOD AND KIT FOR DETECTING PRESENCE AND/OR AMOUNT OF BACTERIA IN FOOD/DRINK SAMPLE, ENVIRONMENTAL SAMPLE, OR BIOLOGICAL SAMPLE**
VERFAHREN UND KIT ZUM NACHWEIS DER ANWESENHEIT UND/ODER DER MENGE VON BAKTERIEN IN EINER LEBENSMITTEL-/GETRÄNKEPROBE, UMWELTPROBE ODER BIOLOGISCHEN PROBE
PROCÉDÉ ET TROUSSE DE DÉTECTION DE LA PRÉSENCE ET/OU DE LA QUANTITÉ DE BACTÉRIES DANS UN ÉCHANTILLON DE NOURRITURE/BOISSON, UN ÉCHANTILLON ENVIRONNEMENTAL OU UN ÉCHANTILLON BIOLOGIQUE

(30) Priority: 15.01.2021 JP 2021004806; 15.01.2021 JP 2021004815
(43) Date of publication of application: 22.11.2023
(73) Proprietor: Asahi Kasei Kabushiki Kaisha, Tokyo 1000006 (JP)
(72) Inventor: TAKAHASHI, Katsuyoshi, Tokyo 100-0006 (JP); ODA, Kotaro, Tokyo 100-0006 (JP); SUGATA, Mika, Tokyo 100-0006 (JP)
(74) Representative: Forstmeyer, Dietmar
(86) International application number: PCT/JP2022/001197
(87) International publication number: WO 2022/154094

(56) References cited:
- EP-A1- 1 104 772
- WO-A1-00/06603
- WO-A1-2010/079739
- WO-A1-2019/243714
- WO-A1-2020/111223
- CN-A- 104 297 474
- JP-A- 2000 088 854
- JP-A- 2001 281 254
- JP-A- 2002 202 309
- JP-A- 2013 164 414
- JP-A- 2017 133 952
- JP-A- 2020 521 127
- JP-A- 2021 156 799
- JP-A- H08 319 297
- KR-A- 20200 070 792
- US-A1- 2006 281 074
- US-A1- 2009 269 789

## Description

### TECHNICAL FIELD

The present invention relates to a method and a kit for detecting the presence and/or amount of bacteria in a food or beverage sample, environmental sample, or biological sample.

### BACKGROUND ART

Conventional methods for determining the degree of contamination of food or beverage samples or environmental samples by bacteria include the culture method (e.g., Patent Literature 1: JP2003-116594 A; and Patent Literature 2: WO2019/142848 A), in which a sample is cultured to check bacterial growth, and the ATP method (e.g., Patent Literature 3: JP-H11-239493 A; and Patent Literature 4: JP2009-136205 A), in which intracellular ATP (adenosine triphosphate) of bacteria in a sample is detected.

However, the conventional culture method requires culture facilities and takes several days to a week before detection, which presents challenges in terms of space, labor, and time. In addition, only specific bacteria matching the culture conditions (e.g., medium composition, aerobic/anaerobic atmosphere, and temperature setting) could grow, making it impossible to simultaneously detect bacteria of a wide spectrum of genera. On the other hand, the conventional ATP method has the problem that it is difficult to identify bacteria from eukaryotic cells derived from foods and beverages, environment, and living organisms, because ATP is present not only in bacterial cells but also in eukaryotic cells (e.g., animal cells and plant cells).

There are actual situations in which the presence and/or amount of bacteria in a food or beverage sample, environmental sample, or biological sample should be detected, such as in bacterial tests for food or beverage and environmental sanitation, where it is necessary to detect bacteria of plural genera comprehensively and rapidly, rather than detecting various bacteria individually (e.g., total bacteria counting, general bacteria counting, detection of coliform group, and detection of Enterobacteriaceae bacteria). Therefore, if a method is provided that can collectively detect plural genera of bacteria present in food or beverage, environment, and living organisms, it will be of high technical significance and utility.

### LIST OF CITATIONS

### Patent Literature

[Patent Literature 1] JP-S63-233774 A
[Patent Literature 2] WO2019/142848 A
[Patent Literature 3] JP-H11-239493 A
[Patent Literature 4] JP2009-136205 A

### SUMMARY OF INVENTION

### PROBLEM TO BE SOLVED BY THE INVENTION

An objective of an embodiment of the present invention is to provide a method and a kit capable of detecting the presence and/or amount of bacteria in food or beverage samples, environmental samples, and biological samples easily and efficiently in a short period of time.

### MEANS TO SOLVE THE PROBLEM

As a result of intensive study, the present inventors have found that simultaneous detection of the presence and/or amount of different bacteria of plural genera in a sample based on antigen-antibody reaction makes it possible to detect the presence and/or amount of bacteria in a sample easily and efficiently in a short period of time. The present inventors have also found that by using a generic antibody that causes antigen-antibody reactions widely with a broad spectrum of bacteria in combination with one or more specific antibodies that cause antigen-antibody reactions specifically with particular bacteria, it is possible to distinguish plural species of bacteria from other components in a sample and detect the presence and/or amount of the bacteria quickly and conveniently. The present inventors have then actually produced antibodies that cause antigen-antibody reactions with plural genera of bacteria and have demonstrated that these antibodies can be used to detect the presence and/or amount of the bacteria in a sample easily and efficiently in a short period of time, thereby arriving at the present invention.

### EFFECT OF THE INVENTION

The method according to the present invention makes it possible to detect the presence and/or amount of bacteria in food or beverage samples, environmental samples, and biological samples easily and efficiently in a short period of time.

### BRIEF EXPLANATION OF FIGURES

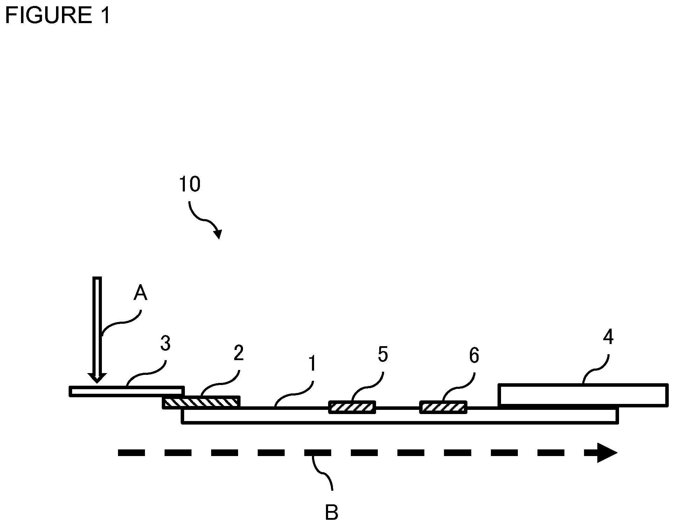

[Figure 1] Figure 1 is a cross-sectional view of a schematic diagram of a strip-shaped detection mechanism, an example of a detection mechanism of a lateral flow type immunochromatographic detection system.

### DESCRIPTION OF EMBODIMENTS

The present invention will be described in detail by referring to the specific embodiments below. However, the present invention should not be limited to the following embodiments, whereas the scope of protection is defined by the appended claims.

In the amino acid sequences described herein, each amino acid shall be represented by a single-letter code, unless otherwise specified.

One embodiment of the present invention relates to in a food or beverage sample, environmental sample, or biological sample a method for detecting the presence and/or amount of bacteria (hereinafter also referred to as "the method of the present invention"). The method of the present invention includes simultaneously detecting plural genera of the presence and/or amount of bacteria in the sample based on antigen-antibody reactions. According to one embodiment, such detection based on antigen-antibody reactions may be carried out by, e.g., contacting the sample with an antibody that causes antigen-antibody reactions with components derived from plural genera of bacteria in the sample (hereinafter also referred to as "the antibody of the present invention"), and measuring the presence and/or intensity of the antigen-antibody reactions that occur in the sample after contact. The method of the present invention makes it possible to determine the degree of contamination of, e.g., a food or beverage sample, environmental sample, or biological sample by bacteria easily and efficiently in a short period of time.

A kit comprising the antibody as recited in the present invention for carrying out the method of the present invention (the kit of the present invention) constitutes another subject of the present invention.

In the following description, the method of the present invention will be explained first, followed by a description of the antibody as recited in the present invention used in the method of the present invention, then by a description of a particularly preferred embodiment of the method of the present invention (the method (2) of the present invention), and lastly by a description of the kit for use in the method of the present invention.

### [1. Method for Detecting the Presence and/or Amount of Bacteria in Sample (1)]

The method of the present invention is a method for detecting the presence and/or amount of bacteria in a sample selected from food or beverage samples, environmental samples, and biological samples. The method of the present invention includes simultaneously detecting the presence and/or amount of bacteria of plural genera in the sample based on antigen-antibody reactions. The method of the present invention may also be used for, e.g., determining the degree of contamination of the sample by bacteria.

Examples of samples include food or beverage samples, environmental samples, and biological samples (hereinafter, food or beverage samples and environmental samples may be referred to collectively as "food, beverage, or environmental samples," and food or beverage samples, environmental samples, and biological samples may be referred to collectively as "food, beverage, environmental, or biological samples"). The types of food or beverage samples are not particularly limited. Examples include samples obtained from food ingredients such as meat, fish, vegetables, processed foods and beverages, and seasonings, and beverages such as water, tea, coffee, juice, and alcoholic beverages. The types of environmental samples are not particularly limited, but examples include samples obtained by wiping surfaces such as fingers, work clothes, work shoes, nail brushes, cutting boards, knives, handles, conveyor belts, packaging materials, work desks, beds, faucets, showers, and medical equipment in environments such as food and beverage manufacturing facilities, food and beverage serving sites, medical devices, medical equipment, and medical care sites with a collection tool (swab) such as clean cotton or clean cloth impregnated with a liquid medium (e.g., water, physiological isotonic solution, ethanol, etc.), as well as liquid samples such as tap water, well water, and river or hot spring water. The types of biological samples are also not particularly limited, but examples include samples derived from human or non-human animals, such as whole blood, serum, plasma, urine, stool, hands, saliva, sputum, sweat, nasal discharge, throat swab, nasal aspirate, and lung lavage fluid. Such food, beverage, environmental, or biological samples can be contaminated by a wide variety of bacteria.

As mentioned above, the culture method and the ATP method have conventionally been used to detect contamination of such food, beverage, environmental, or biological samples by bacteria. However, the culture method requires culture facilities and takes several days to a week before detection. The ATP method has difficulty in identifying bacteria from eukaryotic cells derived from foods and beverages, environment, and living organisms, because ATP is present not only in bacteria but also in eukaryotic cells (e.g., animal cells and plant cells).

The method of the present invention includes simultaneously detecting plural genera of the presence and/or amount of bacteria in the sample based on antigen-antibody reactions. The term "antigen-antibody reaction" used herein refers to the specific binding of an antibody to its antigen. The use of antigen-antibody reactions makes it possible to detect detecting the presence and/or amount of bacteria in the sample immediately on site using simple equipment and operation. The phrase "simultaneous" detection of plural species or plural genera of bacteria herein refers to plural species or plural genera of bacteria at once, and should not necessarily be limited to simultaneous detection in time. According to the present invention, by appropriately selecting and using antibodies (antibodies of the invention) that specifically generate antigen-antibody reactions with components derived from plural genera of bacteria to be detected, it is possible to simultaneously detect the plurality of genera of bacteria to be detected, while reducing false positives caused by components other than bacteria, thereby improving detection sensitivity and detection accuracy. The "simultaneous" detection of bacteria of two or more genera includes not only the final detection of bacteria of two or more genera, but also the detection using antibodies (i.e., generic antibodies described below) that collectively (i.e., "simultaneously") cause antigen-antibody reactions with bacteria of two or more genera, even if the bacteria finally detected belong to a single genus. According to the present invention, by appropriately selecting and using antibodies (antibodies as recited in the invention) that specifically generate antigen-antibody reactions with components derived from plural genera of bacteria to be detected, it is possible to simultaneously detect the plurality of genera of bacteria to be detected, while reducing false positives caused by components other than bacteria, thereby improving detection sensitivity and detection accuracy. As a result, the degree of contamination of the sample by plural genera of can be determined easily and efficiently in a short period of time.

Examples of bacteria to be detected in the present invention include bacteria belonging to the genus Escherichia, the genus Staphylococcus, the genus Pseudomonas, the genus Bacillus, the genus Klebsiella, the genus Serratia, the genus Rahnella, the genus Citrobacter, the genus Listeria, the genus Enterobacter, and the genus Salmonella (hereinafter also referred to as "specific genera"), which are representative bacteria genera with particularly high demand for detection in food, beverage, environmental, or biological samples. According to the method of the present invention, it may be preferable to detect antigen-antibody reactions with bacteria of at least two or more of these specific bacteria genera (plural genera), and especially preferable to detect antigen-antibody reactions with bacteria of at least three, or at least four, or at least five, or at least six, or at least seven, or at least eight, or at least nine, or at least ten, or all of these eleven genera.

Examples of bacteria belonging to the genus Escherichia include Escherichia coli (E. coli, EC) and Escherichia albertii (E. albertii). When the method of the present invention is used for detecting antigen-antibody reactions with bacteria belonging to the genus Escherichia, it is sufficient to detect antigen-antibody reactions with any one or more of these bacteria, but it may be preferred to detect antigen-antibody reactions with at least Escherichia coli (E. coli, EC).

Examples of bacteria belonging to the genus Staphylococcus include Staphylococcus aureus (S. aureus, SA), Staphylococcus epidermidis (S. epidermidis), and Staphylococcus argenteus (S. argenteus). When the method of the present invention is used for detecting antigen-antibody reactions with bacteria belonging to the genus Staphylococcus, it is sufficient to detect antigen-antibody reactions with any one or more of these bacteria, but it may be preferred to detect antigen-antibody reactions with at least Staphylococcus aureus (S. aureus, SA).

Examples of bacteria belonging to the genus Pseudomonas include Pseudomonas aeruginosa (P. aeruginosa, PA), Pseudomonas fluorescens (P. fluorescens), Pseudomonas phosphorescence (P. phosphorescence), and Pseudomonas putida (P. putida). When the method of the present invention is used for detecting antigen-antibody reactions with bacteria belonging to the genus Pseudomonas, it is sufficient to detect antigen-antibody reactions with any one or more of these bacteria, but it may be preferred to detect antigen-antibody reactions with at least Pseudomonas aeruginosa (P. aeruginosa, PA).

Examples of bacteria belonging to the genus Bacillus include Bacillus subtilis (B. subtilis, BS), Bacillus cereus (B. cereus), Bacillus licheniformis (B. licheniformis), and Bacillus megaterium (B. megaterium). When the method of the present invention is used for detecting antigen-antibody reactions with bacteria belonging to the genus Bacillus, it is sufficient to detect antigen-antibody reactions with any one or more of these bacteria, but it may be preferred to detect antigen-antibody reactions with at least Bacillus subtilis (B. subtilis, BS).

Examples of bacteria belonging to the genus Klebsiella include Klebsiella pneumoniae (K. pneumoniae, KP), Klebsiella oxytoca (K. oxytoca), and Klebsiella aerogenes (K. aerogenes). When the method of the present invention is used for detecting antigen-antibody reactions with bacteria belonging to the genus Klebsiella, it is sufficient to detect antigen-antibody reactions with any one or more of these bacteria, but it may be preferred to detect antigen-antibody reactions with at least Klebsiella pneumoniae (K. pneumoniae, KP).

Examples of bacteria belonging to the genus Serratia include Serratia liquefaciens (S. liquefaciens, SL), Serratia marcescens (S. marcescens), and Serratia fonticola (S. fonticola). When the method of the present invention is used for detecting antigen-antibody reactions with bacteria belonging to the genus Serratia, it is sufficient to detect antigen-antibody reactions with any one or more of these bacteria, but it may be preferred to detect antigen-antibody reactions with at least Serratia liquefaciens (S. liquefaciens, SL).

Examples of bacteria belonging to the genus Rahnella include Rahnella aquatilis (R. aquatilis, RA), Rahnella victoriana (R.victoriana), and Rahnella bruchi (R. bruchi). When the method of the present invention is used for detecting antigen-antibody reactions with bacteria belonging to the genus Rahnella, it is sufficient to detect antigen-antibody reactions with any one or more of these bacteria, but it may be preferred to detect antigen-antibody reactions with at least Rahnella aquatilis (R. aquatilis, RA).

Examples of bacteria belonging to the genus Citrobacter include Citrobacter freundii (C. freundii, CF), Citrobacter amalonaticus (C. amalonaticus), and Citrobacter diversus (C. diversus). When the method of the present invention is used for detecting antigen-antibody reactions with bacteria belonging to the genus Citrobacter, it is sufficient to detect antigen-antibody reactions with any one or more of these bacteria, but it may be preferred to detect antigen-antibody reactions with at least Citrobacter freundii (C. freundii, CF).

Examples of bacteria belonging to the genus Listeria include Listeria monocytogenes (L. monocytogenes, LM), Listeria innocua (L. innocua), and Listeria ivanovii (L. ivanovii). When the method of the present invention is used for detecting antigen-antibody reactions with bacteria belonging to the genus Listeria, it is sufficient to detect antigen-antibody reactions with any one or more of these bacteria, but it may be preferred to detect antigen-antibody reactions with at least Listeria monocytogenes (L. monocytogenes, LM).

Examples of bacteria belonging to the genus Enterobacter include Enterobacter cloacae (E. cloacae, ECL), Enterobacter aerogenes (E. aerogenes), and Enterobacter sakazakii (E. sakazakii). When the method of the present invention is used for detecting antigen-antibody reactions with bacteria belonging to the genus Enterobacter, it is sufficient to detect antigen-antibody reactions with any one or more of these bacteria, but it may be preferred to detect antigen-antibody reactions with at least Enterobacter cloacae (E. cloacae, ECL).

Examples of bacteria belonging to the genus Salmonella include Salmonella enteritidis (S. enteritidis, SE), Salmonella infantis (S. infantis), and Salmonella typhimurium (S. typhimurium). When the method of the present invention is used for detecting antigen-antibody reactions with bacteria belonging to the genus Salmonella, it is sufficient to detect antigen-antibody reactions with any one or more of these bacteria, but it may be preferred to detect antigen-antibody reactions with at least Salmonella enteritidis (S. enteritidis, SE).

Examples of the genera of bacteria that can be detected by the method of the present invention include, although are not limited to, the genus Enterococcus, the genus Moraxella, the genus Aeromonas, the genus Lactobacillus, the genus Micrococcus, the genus Acinetobacter, the genus Campylobacter, the genus Proteus, and the genus Vibrio.

According to one embodiment of the present invention, the bacteria to be detected may include bacteria belonging to Enterobacteriaceae (hereinafter also referred to as "Enterobacteriaceae bacteria"). Examples of Enterobacteriaceae bacteria with particularly high demand for detection in the present embodiment include, although are not limited to, those belonging to the genus Escherichia, the genus Klebsiella, the genus Citrobacter, the genus Enterobacter, the genus Proteus, the genus Salmonella, and the genus Serratia, which are representative Enterobacteriaceae genera with particularly high demand for detection in food, beverage, environmental, or biological samples. Examples of other Enterobacteriaceae bacteria that can be detected in the present embodiment include, although are not limited to, those belonging to the genus Yersinia, the genus Erwinia, the genus Hafnia, the genus Morganella, the genus Obesumbacterium, the genus Providencia, the genus Shigella, the genus Aeromonas, and the genus Pectobacterium.

According to one embodiment, the plurality of genera of bacteria may preferably include both one or more Gram-negative bacteria and one or more Gram-positive bacteria. Because the cell membrane and cell wall structures are very different between these groups, it is difficult to detect both groups of bacteria simultaneously using conventional technology. On the other hand, the method of the present invention allows for simultaneous detection of Gram-negative bacteria and Gram-positive bacteria, provided that the antibodies used for detection are appropriately designed.

According to the method of the present invention, compared to the conventional methods such as the culture method and the ATP method, it is possible to detect the presence and/or amount of bacteria in food, beverage, environmental, or biological samples more easily and efficiently in a shorter period of time, by simultaneously detecting antigen-antibody reactions with these bacteria. The method of the present invention also makes it possible, for example, to determine the degree of contamination of food, beverage, environmental, or biological samples with bacteria much more quickly and easily.

### [2. Antibodies that Cause Antigen-Antibody Reactions with Components Derived from Multiple Genera of Bacteria in Sample]

There are no restrictions to the means to be employed in the method of the present invention for simultaneously detecting the presence and/or amount of bacteria of plural genera in the sample based on antigen-antibody reactions. According to one embodiment, such detection based on antigen-antibody reactions may preferably be carried out by contacting the sample with an antibody that causes antigen-antibody reactions with components derived from bacteria and measuring the presence and/or intensity of the antigen-antibody reactions that occur in the sample after contact. The antibody will be explained below

The term "antibody" used herein refers to a protein that recognizes and binds to a specific antigen or substance, which may also be referred to as an immunoglobulin (Ig). Common antibodies typically have two light chains (light chains) and two heavy chains (heavy chains) that are interconnected by disulfide bonds. There are two classes of light chains, called λ and κ chains, and five classes of heavy chains, called γ, µ, α, δ, and ε chains. Depending on the class of their heavy chains, antibodies are classified into five isotypes: IgG, IgM, IgA, IgD and IgE, respectively.

Heavy chains each include a heavy chain constant (CH) region and a heavy chain variable (VH) region. Light chains each include a light chain constant (CL) region and a light chain variable (VL) region. The light chain constant (CL) region consists of a single domain. The heavy chain constant (CL) region consists of three domains, namely CH1, CH2, and CH3. The light chain variable (VL) region and the heavy chain variable (VH) region each consist of four highly conserved regions called framework regions (FRs; FR-1, FR-2, FR-3, and FR-4) and three hypervariable regions called complementarity-determining regions (CDRs; CDR-1, CDR-2, and CDR-3). The heavy chain constant (CH) region consists of three CDRs (CDR-H1, CDR-H2, and CDR-H3) and four FRs (FR-H1, FR-H2, FR-H3, and FR-H4), which are arranged from the amino terminus to the carboxy terminus in the order of FR-H1, CDR-H1, FR-H2, CDR-H2, FR-H3, CDR-H3, and FR-H4. The light chain constant (CL) region has three CDRs (CDR-L1, CDR-L2, CDR-L3) and four FRs (FR-L1, FR-L2, FR L3, and FR-L4), which are arranged from the amino terminus to the carboxy terminus in the order of FR-L1, CDR-L1, FR-L2, CDR-L2, FR-L3, CDR-L3, and FR-L4. The variable regions of the heavy and light chains contain binding domains that interact with the antigen.

The antibody may be either a polyclonal antibody or a monoclonal antibody, but a monoclonal antibody be preferred. A polyclonal antibody is usually prepared from the serum of an animal immunized with an antigen and is a mixture of various antibody molecules with different structures. A monoclonal antibody, on the other hand, is an antibody composed of a single type of molecules containing a combination of light chain variable (VL) and heavy chain variable (VH) regions having determined amino acid sequences. Monoclonal antibodies can be produced from clones derived from antibody-producing cells, or they can be produced using genetic engineering technique, by obtaining nucleic acid molecules having gene sequences encoding amino acids of antibody proteins. It is also a well-known technique to those skilled in the art to improve the binding and specificity of antibodies using genetic information of their heavy chains and light chains or their variable regions and CDRs.

The antibody may be a fragment and/or derivative of an antibody. Fragments of antibodies include F(ab')₂, Fab, Fv, etc. Antibody derivatives include antibodies in which amino acid mutations have been artificially introduced into the constant region(s) of the light and/or heavy chains, antibodies in which the domain configuration of the constant region(s) of the light and/or heavy chains has been modified, antibodies with two or more Fc regions per molecule, glycosylated antibodies, bispecific antibodies, antibody conjugates in which an antibody or antibody fragment is bound to a protein other than the antibody, antibody enzymes, antibody conjugates in which an antibody or antibody fragment is bound to a protein other than an antibody, etc. Antibody conjugates, antibody enzymes, tandem scFv, bispecific tandem scFv, diabody, etc. Furthermore, when the aforementioned antibodies or their fragments or derivatives are derived from non-human animals, chimeric or humanized antibodies in which some or all of the sequences other than the CDRs thereof are replaced with the corresponding sequences of human antibodies are also disclosed. When the simple term "antibodies" is used herein, it is intended to also encompass fragments and/or derivatives of antibodies, unless otherwise specified.

When the antibody causes antigen-antibody reactions with certain bacteria, it means that they bind specifically to some components of the bacteria as antigens. The components of bacteria that serve as antigens for the antibody are not limited. It may be a component contained in the cell walls or cell membranes that are exposed outside the bacterial cells, or it may be a component contained in the cytoplasm, cell organelles, or nucleus and not exposed outside the bacterial cells. When the antibody causes antigen-antibody reactions with components of bacteria that are not exposed outside the cell surface of the bacteria, the food, beverage, environmental, or biological sample may be subjected to a treatment for lysing the bacteria before being brought into contact with the antibody. Such bacterial lysis treatment will be described later.

The antibody may preferably cause antigen-antibody reactions with bacteria belonging to 5 or more, or 6 or more, or 7 or more, or 8 or more, or 9 or more, or 10 or more, or 11 or more genera selected from the group consisting of the genus Escherichia, the genus Staphylococcus, the genus Pseudomonas, the genus Bacillus, the genus Klebsiella, the genus Serratia, the genus Rahnella, the genus Citrobacter, the genus Listeria, the genus Enterobacter, and the genus Salmonella. The specific bacterial species belonging to these genera are described above.

The antibody may preferably cause antigen-antibody reactions with ribosome proteins, and more preferably with ribosome proteins L7/L12, of bacteria belonging to any of the specific bacteria genera and/or selective bacteria genera mentioned above. As used herein, the term "ribosome protein L7/L12," or simply "L7/L12," refers to a type of ribosomal protein essential for microbial protein synthesis and commonly possessed by various bacteria. For antibodies that cause antigen-antibody reactions against the bacterial ribosomal proteins L7/L12 and methods for producing the same, reference may be made to, e.g., to WO2000/006603A, the publication of an earlier patent application filed by the present inventors.

The components of bacteria that serve as antigens for the antibody are not limited. They may be components contained in the cell walls or cell membranes that are exposed outside the bacterial cells, or they may be components that are contained in the cytoplasm, cell organelles, or nucleus and not exposed outside the bacterial cells. When the antibody causes antigen-antibody reactions with components of bacteria that are not exposed outside the cell surface of the bacteria, the food, beverage, environmental, or biological sample may be subjected to a treatment for lysing the bacteria before being brought into contact with the antibody of the present invention. Such bacterial lysis treatment will be described later.

The degree of antigen-antibody reactions between the antibodies and bacteria is not particularly limited, but it is sufficient that at least antigen-antibody reactions occur to the extent that it can be detected by any known detection method. Methods for detecting antigen-antibody reactions between antibodies and bacteria are not limited, but include various known immunological assays as described below.

The antibody may also preferably not cause any cross-reactions with one or more non-bacterial components that may be present in the sample. Examples of such non-bacterial components include, although are not limited to, various bioorganic compounds derived from viruses, plants, and/or animals that are not present in bacteria. Specific examples of such bioorganic compounds include proteins, sugars, glycoproteins, lipids, complex lipids, and nucleic acids. The antibody may preferably not cause any cross-reactions with components derived from at least one, or 2 or more, or 3 or more, or 4 or more, or 5 or more, or 6 or more, or 7 or more, or 8 or more, especially 9 or more, most preferably 10 or more, of these non-bacterial organic compounds.

Because antibodies are extremely antigen-specific, they are effective in specifically capturing certain antigens, but were considered unsuitable for, e.g., detecting plural different target substances. Moreover, since bacterial species that need to be detected in food or beverage tests are extremely diverse, it has conventionally been considered extremely difficult to simultaneously detect plural species of bacteria by antigen-antibody reactions. However, as will be described in the Examples below, the present inventors have succeeded in obtaining antibodies that cause antigen-antibody reactions with plural species of bacteria that can be the target for detection when testing food or beverage samples or environmental samples, and that can be used for simultaneous detection of these bacteria. This is an extremely surprising finding that goes against conventional technical knowledge.

The structure of the antibody is not particularly limited but may preferably be as explained below. Incidentally, antibodies defined solely by the structural features described below shall also be included in the specification.

Specifically, the antibody may have any of the amino acid sequences mentioned below as the amino acid sequences of each variable region of the heavy and light chains.

The VH sequence is an amino acid sequence selected from SEQ ID NO:1, SEQ ID NO:3, and SEQ ID NO: 5.

The VL sequence is an amino acid sequence selected from SEQ ID NO:2, SEQ ID NO:4, and SEQ ID NO:6.

Combinations of a heavy chain variable region (VH) having a homology (preferably identity) of 100%, with the amino acid sequence of SEQ ID NO: 1 and a light chain variable region (VL) having a homology (preferably identity) 100%, with the amino acid sequence of SEQ ID NO:2.

Combinations of a heavy chain variable region (VH) having a homology (preferably identity) of 100%, with the amino acid sequence of SEQ ID NO:3 and a light chain variable region (VL) having a homology (preferably identity) of 100%, with the amino acid sequence of SEQ ID NO:4.

Combinations of a heavy chain variable region (VH) having a homology (preferably identity) of 100%, with the amino acid sequence of SEQ ID NO:5 and a light chain variable region (VL) having a homology (preferably identity) 100%, with the amino acid sequence of SEQ ID NO:6.

As used herein, the term "homology" between two amino acid sequences refers to the ratio in which identical or similar amino acid residues appear in each corresponding position when these amino acid sequences are aligned, and the term "identity" between two amino acid sequences refers to the ratio of similar amino acid residues appearing in each corresponding position when these amino acid sequences are aligned. The "homology" and "identity" between two amino acid sequences can be determined using, for example, the BLAST (Basic Local Alignment Search Tool) program (Altschul et al., J. Mol. Biol., (1990), 215(3):403-10). (1990, 215(3):403-10).

Methods for identifying the sequence of each CDR from the respective variable sequences of the heavy and light chains of an antibody include, for example, the Kabat method (Kabat et al., The Journal of Immunology, 1991, Vol. 147, No. 5, pp. 1709-1719) and the Chothia method (Al Lazikani et al., Journal of Molecular Biology, 1997, Vol. 273, No. 4, pp. 927-948). These methods are common knowledge in this field, but it is possible to refer to, for example, the website of Dr. Andrew C.R. Martin's Group (http://www.bioinf.org.uk/abs/).

Similar amino acids include, for example, amino acids that are classified under the same group in the following classification based on the polarity, charge, and size of each amino acid (in any case, each amino acid type is indicated by a single-letter code).
*Aromatic amino acids: F, H, W, Y;
*Aliphatic amino acids: I, L, V;
*Hydrophobic amino acids: A, C, F, H, I, K, L, M, T, V, W, Y;
*Charged amino acids: D, E, H, K, R, etc.;
*Positively charged amino acids: H, K, R;
*Negatively charged amino acids: D, E;
*Polar amino acids: C, D, E, H, K, N, Q, R, S, T, W, Y;
*Small amino acids: A, C, D, G, N, P, S, T, V, etc.; and
*Micro amino acids: A, C, G, S.

Similar amino acids also include, for example, amino acids that are classified under the same group in the following classification based on the side chain of each amino acid (in any case, each amino acid type is indicated by a single-letter code).
*Amino acids having an aliphatic side chain: G, A, V, L, I;
*Amino acids having an aromatic side chain: F, Y, W;
*Amino acids having a sulfur-containing side chain: C, M;
*Amino acids having an aliphatic hydroxyl side chain: S, T;
*Amino acids having a basic side chain: K, R, H;
*Amino acids having an acidic side chain and their amide derivatives: D, E, N, Q.

The method of producing the antibody is not restricted. In the case of polyclonal antibodies, they can be prepared using components derived from bacteria to be detected. The components derived from that can be used include bacterial cells, lysate made by lysing bacterial cells, and a fraction of lysate obtained via electrophoresis. When an electrophoretic fraction of bacterial lysate is used, there are no restrictions on which fraction should be used, but it is preferable to select a fraction that corresponds to a molecular weight of approximately 10 to 20 kDa, for example. In addition, it is preferable to use ribosomal proteins contained in bacteria as components derived from bacteria, especially ribosomal proteins L7/L12. These bacteria-derived components are inoculated into an animal, optionally together with adjuvants as necessary, and the serum is collected to obtain antiserum containing antibodies (polyclonal antibodies) that causes antigen-antibody reactions with the plural species of bacteria mentioned above. Examples of animals to be inoculated include sheep, horses, goats, rabbits, mice, rats, etc., of which sheep and rabbits are especially preferred for polyclonal antibody production. The antibodies may be purified and fractionated from the obtained antiserum and screened for antigen-antibody reactivity with the desired bacteria, and for no cross-reactivity with other components of food or beverage, environment, or biological origin, using known methods to obtain the desired antibodies with superior specificity. Furthermore, monoclonal antibodies can be obtained by isolating antibody-producing cells that produce desired antibody molecules and fusing them with myeloma cells to produce hybridomas capable of autonomous growth. As a method that does not require sensitization of animals, a phage library expressing heavy-chain variable (VH) regions or light-chain variable (VL) regions of antibodies or parts thereof can be screened for phage clones expressing specific amino acid sequences that specifically bind to components derived from bacteria to be detected, and antibodies can be produced using information of the screened phage clones.

Once the desired antibodies are obtained by the above procedure, the structure of each antibody, specifically part or all of the amino acid sequences of the heavy chain constant (CH) region, heavy chain variable (VH) region, light chain constant (CL) region, and/or light chain variable (VL) region, can be analyzed using known amino acid sequence analysis methods. It is also known to those skilled in the art to modify the amino acid sequences of the desired antibodies thus obtained to improve their binding ability and specificity. Furthermore, it is also possible to design other antibodies likely to have similar antigen specificity by using all or part of the amino acid sequences of each desired antibody (especially the amino acid sequences of all or part of the heavy chain variable (VH) region and the light chain variable (VL) region, especially the amino acid sequence of each CDR) and, if necessary, by combining them with part of amino acid sequences of known antibodies (especially the amino acid sequences of each FR of the heavy chain variable (VH) and light chain variable (VL) regions, and optionally of the heavy chain variable (VH) and light chain variable (VL) regions).

Once the amino acid sequences of a desired antibody are determined, a nucleic acid molecule having base sequences encoding all or part of the amino acid sequences of the desired antibody can be produced by a known method, and the antibody can be produced by genetic engineering using such a nucleic acid molecule. Furthermore, it is also possible to create a vector or plasmid for expressing each component of the desired antibody from such a nucleic acid sequence, and introduce it into a host cell (e.g., a mammalian cell, insect cell, plant cell, yeast cell, or microbial cell) to produce the antibody. In addition, modifications can be made to the structures of the constant regions of the antibody or to its sugar chains in order to improve the efficiency of the obtained antibody or to avoid its side effects, using techniques well known to those skilled in the art.

Those explained above, i.e., methods for producing the antibody, nucleic acid molecules encoding the antibody of the present invention, vectors or plasmids containing such nucleic acid molecules, cells containing such nucleic acid molecules, vectors or plasmids, hybridomas producing the antibodies, etc., are also disclosed herein.

The techniques for making and modifying the antibodies described herein are all known to those skilled in the art, and may be carried out by referring to, e.g., Antibodies; A laboratory manual, E. Harlow et al. Likewise, the molecular biological techniques described herein (e.g., amino acid sequencing methods, methods for designing and producing nucleic acid molecules, methods for designing and producing vectors and plasmids, etc.) are also all known to those skilled in the art, and may be carried out by referring to, e.g., Molecular Cloning, A laboratory manual, Cold Spring Harbor Laboratory Press, Shambrook, NY. Harbor Laboratory Press, Shambrook, J. et al.

Thus, a preferred embodiment of the method of the present invention includes using an antibody that recognizes ribosomal proteins of each bacterium and causes antigen-antibody reactions (the antibody of the present invention), bringing them into contact with the sample, and detecting antigen-antibody reactions. In this embodiment, the detection sensitivity can be improved by exposing the ribosomal proteins of bacteria present in the sample to outside the bacterial cell membranes before bringing the sample into contact with the antibody. Thus, the sample may preferably be subjected to treatment for lysing bacteria before being brought into contact with the antibody. Examples of the lysis treatment of bacteria include, although are not limited to, heating treatment, ultrasonication, and chemical treatment using a surfactant. Conditions for the lysis treatment may be determined as appropriate depending on the bacteria contained in the sample. The may be brought into contact with the food, beverage, environmental, or biological sample by any arbitrary means.

Immunological assays for detecting antigen-antibody reactions are not limited. Examples of immunological assays are not limited and may be either methods using a single antibody or those using two or more antibodies.

Examples of immunological assays using a single antibody include, although are not limited to, various known immunological assays such as ELISA (enzyme-linked immunosorbent assay) using microtiter plates loaded with bacterial antigens to detect antigen-antibody reactions with antibodies; and biosensors that have antibodies (or antigens) loaded on the sensor surface to detect antigen-antibody reactions with antigens (or antibodies) electrically (e.g., AC impedance method, FET (field effect transistor) method) or optically (SPR (surface plasmon resonance) method). Any of these assays can be used in the method of the present invention.

Examples of immunological assay methods using two or more antibodies include, although are not limited to, various known immunological assays such as ELISA using antibody-loaded microtiter plates; latex particle agglutination assay using latex particles (e.g. polystyrene latex particles) loaded with antibodies; immunochromatography using antibody-loaded membranes; and sandwich assay using a detection antibody labeled with colored or chromogenic particles, enzymes or fluorophores, etc., and a capture antibody immobilized on a solid phase carrier such as magnetic particles, etc. In the case of immunological assays that combine two or more antibodies, such as the sandwich assay, in which a detection antibody and a capture antibody are used, the antibody may be used either as the capture antibody or as the detection antibody. Unless otherwise specified, the term "specific antibodies" as used herein refers to antibodies that cause antigen-antibody reactions with specific bacteria, including two or more species (bacteria to be detected) that are the final targets of detection, while the term "generic antibodies" as used herein refers to antibodies that cause antigen-antibody reactions with bacteria of five or more genera, including the aforementioned bacteria to be detected.

### [3. Method for Detecting the Presence and/or Amount of Bacteria in Sample (2)]

According to the present invention, it may be preferred to use, as a sandwich assay, a method of detecting the presence and/or amount of the bacteria in the sample by capturing the bacteria in the sample and labeling the bacteria in the sample based on antigen-antibody reactions between the sample, a capture antibody bound to a solid-phase carrier, and a detection antibody having a detection label, and detecting the bacteria to be detected in the sample based on the detection label. This method (hereinafter also referred to as "the method (2) of the present invention") will be explained below.

The method of the present invention (2) is a method for detecting the presence and/or amount of bacteria in the sample by (I) capturing the bacteria in the sample and detecting the bacteria in the sample based on antigen-antibody reactions between the sample, a capture antibody bound to a solid-phase carrier, and a detection antibody having a detection label, and (II) detecting the bacteria to be detected in the sample based on the detection label. This method is further characterized in that one of the capture antibody and the detection antibody is at least one specific antibody, which causes antigen-antibody reactions with one or more bacteria to be detected, and the other is bacteria of five or more genera including the bacteria to be detected causes antigen-antibody reactions with, at least one generic antibody.

One embodiment of the method (2) of the present invention (hereinafter referred to as "Embodiment A") may be characterized in that Step (I) includes the steps of:
(Ia-1) contacting the sample with the detection antibody and detecting the bacteria in the sample based on antigen-antibody reactions between the detection antibody and bacteria, and
(Ia-2) contacting the capture antibody with the sample containing the bacteria labeled by the detection antibody and capturing the bacteria in the sample based on antigen-antibody reactions between the capture antibody and the bacteria-detection antibody complex.

Another embodiment of the method (2) of the present invention (hereinafter referred to as "Embodiment B") may be characterized in that Step (I) includes the steps of:
(Ib-1) contacting the sample with the capture antibody and capturing the bacteria in the sample based on antigen-antibody reactions between the capture antibody and bacteria, and
(Ib-2) contacting the detection antibody with the sample containing the bacteria captured by the capture antibody and detecting the bacteria in the sample based on antigen-antibody reactions between the detection antibody and the bacteria-capture antibody complex.

In either embodiment, the capture antibody may be the generic antibody and the detection antibody may be the specific antibody. Alternatively, the detection antibody may be the generic antibody and the capture antibody may be the specific antibody. In either case, the antibody may be either the generic antibody or the specific antibody. The antibody may preferably be, although is not limited to, used as the generic antibody. Unless otherwise specified, the term "specific antibodies" as used herein refers to antibodies that cause antigen-antibody reactions with specific bacteria, including two or more species (bacteria to be detected) that are the final targets of detection, while the term "generic antibodies" as used herein refers to antibodies that cause antigen-antibody reactions with 5 or more, or 6 or more, or 7 or more, or 8 or more, or 9 or more, or 10 or more, or 11 or more genera of bacteria, including the aforementioned bacteria to be detected.

In the method (2) of the present invention, either Embodiment A or Embodiment B may be chosen according to the type of immunoassay method actually used and the type of samples. Examples of immunoassay methods include, although are not limited to, various known immunological assays such as ELISA (enzyme-linked immunosorbent assay) using microtiter plates loaded with bacterial antigens to detect antigen-antibody reactions with antibodies; and biosensors that have antibodies (or antigens) loaded on the sensor surface to detect antigen-antibody reactions with antigens (or antibodies) electrically (e.g., AC impedance method, FET (field effect transistor) method) or optically (SPR (surface plasmon resonance) method).

The following explanation will be given based on an example where Embodiment A of the method (2) of the present invention is carried out using immunochromatography as the immunoassay. Each feature may be modified as appropriate when other types of immunoassay methods are used.

In Step (Ia-1) above, i.e., the step of contacting the sample with the detection antibody and labeling the bacteria in the sample based on antigen-antibody reactions between the detection antibody and bacteria, the detection antibody with the label for detection is brought into contact with the sample, so that the bacteria in the sample are labeled based on antigen-antibody reactions between the detection antibody and the bacteria. The method for bringing the sample into contact with the detection antibody is not limited, but this may typically be carried out by introducing the sample prepared as an aqueous sample onto a member area impregnated with the detection antibody, and maintaining it for a certain period of time. Although this can be achieved by a variety of specific embodiments depending on, e.g., the mode of capture in Step (a), one example includes preparing a solid-phase carrier (porous membrane) on which the capture antibody is immobilized, preparing a conjugate pad to which the detection antibody is attached, placing the conjugate pad upstream of the solid-phase carrier, and introducing the sample prepared as an aqueous sample onto the conjugate pad and allowing it to pass through, thereby bringing the sample into contact with the detection antibody. As another example, when a flow channel is used as the solid-phase carrier, the sample prepared as an aqueous sample may be brought into contact with the detection antibody either upstream of the position on the flow channel where the capture antibody is immobilized or before the introduction of the solid-phase carrier.

In Step (Ia-2) above, i.e., the step of contacting the capture antibody with the sample containing the bacteria labeled by the detection antibody and capturing the bacteria in the sample based on antigen-antibody reactions between the capture antibody and the bacteria-detection antibody complex, the capture antibody is brought into contact with the sample, so that the bacteria in the sample is captured based on antigen-antibody reactions between the capture antibody and the bacteria. The method for bringing the sample into contact with the capture antibody is not limited, but this may typically be carried out by introducing the sample prepared as an aqueous sample onto an area where the capture antibody is present, and maintaining it for a certain period of time. Although this can be achieved by a variety of specific embodiments depending on, e.g., the type of solid-phase carrier of the capture antibody, one example includes using a porous membrane as the solid-phase carrier, and introducing the bacteria-detection antibody complex onto the porous membrane to which the capture antibody is immobilized and allowing it to permeate, such that the bacteria in the sample is captured by the capture antibody immobilized on the porous membrane. Another example includes immobilizing the capture antibody on an area of a flow channel used as the solid-phase carrier, and allowing the bacteria-detection antibody complex to pass through the flow channel to allow the capture antibody immobilized on the area of the flow channel to capture the bacteria in the sample.

In Step (II) above, i.e., the step detecting the bacteria to be detected in the sample based on the detection label, the bacteria to be detected captured by the capture antibody and labeled with the detection antibody is detected based on the detection label. The detection method is not restricted and may be selected depending on the type of detection label. For example, when a metallic colloid such as gold colloid is used as the detection label, the presence or amount of gold colloid bound to the target bacteria may be detected by any method such as visual inspection or using a camera.

According to the method (2) of the present invention described above, it is possible to distinguish the desired bacteria from other bacteria or other components in the sample and to detect them easily and efficiently, by appropriately combining a generic antibody, which causes antigen-antibody reactions widely with various species of bacteria, and a specific antibody, which causes antigen-antibody reactions specifically with a particular species of bacteria. Specifically, by using an appropriate combination of plural specific antibodies, it is possible to design a system for detecting various desired combinations of bacteria. Any immunoassay method can be used to perform method (2) of the present invention, as long as it employs capture and detection antibodies.

Specifically, when the antibody is used as the generic antibody, it is possible to prepare, as the generic antibody, an antibody that has relatively low specificity, i.e., that causes antigen-antibody reactions with a wide variety of bacteria (usually 5 or more genera), including the bacteria that are the final target of detection, and to use it in combination with, as the specific antibody, an antibody with relatively high specificity, i.e., that causes antigen-antibody reactions only with the bacteria that are the final target of detection, and also does not antigen-antibody reactions with bacteria of other genera.

As mentioned above, the generic antibody may be used either as the capture antibody or as the detection antibody, but may preferably be used as the detection antibody. The detection antibody needs to be labeled for detection, and the labeling conditions (pH, salt concentration, buffer type, etc.) need to be optimized according to the antibody species used. By using the generic antibody as the detection antibody, the same generic antibody can be used even if the bacteria to be detected are changed, eliminating the need to optimize such labeling conditions. This is especially advantageous when producing a kit for sandwich assay.

On the other hand, when the antibody is used as the specific antibody, it is possible to prepare, as the specific antibody, an antibody that has relatively high specificity, i.e., that causes antigen-antibody reactions only with the bacteria of two or more genera that are the final target of detection and does not cause antigen-antibody reactions with bacteria of other genera, and to use it in combination with, as the generic antibody, an antibody with relatively low specificity, i.e., that causes antigen-antibody reactions with a wide variety of bacteria, including the bacteria to be detected as well as other genera with which the antibody does not cause antigen-antibody reactions.

In the method (2) of the present invention, the specific antibody may preferably cause antigen-antibody reactions only with bacteria of limited genera. Specifically, the range of bacteria with which the specific antibody causes antigen-antibody reactions may preferably be commensurate with the range of bacteria to be detected. When only a single specific antibody is used, the range of bacteria for which the specific antibody causes antigen-antibody reactions may be commensurate with the range of bacteria to be detected. On the other hand, when two or more specific antibodies are used in combination, the combined range of bacteria with which the specific antibodies cause antigen-antibody reactions in combination may be commensurate with the range of bacteria to be detected. The latter embodiment is particularly advantageous because it makes it possible to adjust the range of bacteria to be detected in various ways by appropriately combining plural specific antibodies that each cause antigen-antibody reactions with different bacteria.

In the method (2) of the present invention, each specific antibody may cause antigen-antibody reactions with at least one genus of bacteria. The specific bacterial genera with which each specific antibody causes antigen-antibody reactions are not limited, but each specific antibody may preferably cause antigen-antibody reactions at least with bacteria of one or more genera selected from the genus Escherichia, the genus Staphylococcus, the genus Pseudomonas, the genus Bacillus, the genus Klebsiella, the genus Serratia, the genus Rahnella, the genus Citrobacter, the genus Listeria, the genus Enterobacter, and the genus Salmonella.

In the method (2) of the present invention , each generic antibody may preferably cause antigen-antibody reactions at least with bacteria of 5 or more, or 6 or more, or 7 or more, or 8 or more, or 9 or more, or 10 or more, or 11 or more genera selected from the genus Escherichia, the genus Staphylococcus, the genus Pseudomonas, the genus Bacillus, the genus Klebsiella, the genus Serratia, the genus Rahnella, the genus Citrobacter, the genus Listeria, the genus Enterobacter, and the genus Salmonell.

In the method (2) of the present invention , the structures of each specific antibody and each generic antibody are restricted to the claimed sequences.

The VH sequence has the amino acid sequence selected from SEQ ID NO:1, SEQ ID NO:3, and SEQ ID NO:5.

The VL sequence has the amino acid sequence selected from SEQ ID NO:2, SEQ ID NO:4, and SEQ ID NO:6.

Combinations of a heavy chain variable region (VH) having a homology (preferably identity) of 100%, with the amino acid sequence of SEQ ID NO: 1 and a light chain variable region (VL) having a homology (preferably identity) of 100%, with the amino acid sequence of SEQ ID NO:2.

Combinations of a heavy chain variable region (VH) having a homology (preferably identity) of 100%, with the amino acid sequence of SEQ ID NO:3 and a light chain variable region (VL) having a homology (preferably identity) of 100%, with the amino acid sequence of SEQ ID NO:4.

Combinations of a heavy chain variable region (VH) having a homology (preferably identity) of 100%, with the amino acid sequence of SEQ ID NO:5 and a light chain variable region (VL) having a homology (preferably identity) of 100%, with the amino acid sequence of SEQ ID NO:6.

Combinations of a heavy chain variable region (VH) having a homology (preferably identity) of 100%, with the amino acid sequence of SEQ ID NO:7 and a light chain variable region (VL) having a homology (preferably identity) of 100%, with the amino acid sequence of SEQ ID NO:8.

Combinations of a heavy chain variable region (VH) having a homology (preferably identity) of 100%, with the amino acid sequence of SEQ ID NO:9 and a light chain variable region (VL) having a homology (preferably identity) of 100%, with the amino acid sequence of SEQ ID NO:10.

Combinations of a heavy chain variable region (VH) having a homology (preferably identity) of 100%, with the amino acid sequence of SEQ ID NO:11 and a light chain variable region (VL) having a homology (preferably identity) of 100%, with the amino acid sequence of SEQ ID NO: 12.

Combinations of a heavy chain variable region (VH) having a homology (preferably identity) of 100%, with the amino acid sequence of SEQ ID NO:13 and a light chain variable region (VL) having a homology (preferably identity) of 100%, with the amino acid sequence of SEQ ID NO: 14.

### [4. Kit for Detecting the Presence and/or Amount of Bacteria in the Sample]

As mentioned above, a kit for use in the method of the present invention containing the antibody as recited in the present invention (the kit of the invention) is also included in the subject of the present invention.

The kit of the present invention includes, in addition to the antibody, one or more reagents and a detection device or components thereof necessary to perform the method of the present invention using the antibody as recited in the present invention, and/or instructions describing the procedure for performing the method of the present invention. The type of such reagents and instructions, as well as other components included in the kit of the present invention, may be determined according to the specific immunological assay used to detect bacteria of plural genera.

When the kit of the present invention contains a device for detection or components thereof, the device assembled from the kit is a device equipped with components necessary for performing the method of the present invention using the antibody as recited in the present invention. The specific components of the device may be determined according to the type of immunological assay as a specific embodiment of the method of the present invention. As explained above, examples of immunological assay methods using two or more antibodies include, although are not limited to, various known immunological assays such as ELISA using antibody-loaded microtiter plates; latex particle agglutination assay using latex particles (e.g. polystyrene latex particles) loaded with antibodies; immunochromatography using antibody-loaded membranes; and sandwich assay using a detection antibody labeled with colored or chromogenic particles, enzymes or fluorophores, etc., and a capture antibody immobilized on a solid phase carrier such as magnetic particles, ELISA using a single antibody, and biosensor methods. Devices equipped with components necessary to perform such various immunological assays may be used as the device.

Specific examples of devices capable of simultaneously and simply detecting the presence and/or amount of bacteria of plural genera in the sample include devices of the lateral-flow type and those of the flow-through type. According to the lateral-flow devices, the analyte to be detected and the antibody to be detected are deployed parallel onto a membrane having a detection area on the surface of which the capture antibody is immobilized, and the target substance captured in the detection area of the membrane is detected. On the other hand, according to the flow-through devices, the analyte to be detected and the detection antibody are passed vertically through a membrane on which the capture antibody is immobilized on the surface, and the target substance captured on the membrane surface is detected. The method of the present invention can be applied to both lateral-flow devices and flow-through devices.

Both lateral-flow type and flow-through type immunochromatographic detection devices are well known, and the details of such devices can be designed by those skilled in the art based on their technical knowledge, other than those described herein. The following is a description of the schematic configuration of the detection mechanism of the lateral flow type immunochromatographic detection device, with reference to the drawings. The schematic configuration of the detection mechanism of the lateral-flow type immunochromatography detection system will be described below with reference to the figure. However, these are only examples of the schematic configuration of the detection procedure, and the configuration of the lateral-flow type immunochromatographic detection system is not limited in any way to the embodiment illustrated in the figure.

Figure 1 is a cross-sectional view of a schematic configuration of a strip-shaped detection mechanism, which is an example of a detection mechanism of a lateral-flow type immunochromatographic detection system. The detection mechanism 10 of Figure 1 contains an insoluble membrane carrier 1 for chromatographic development; a strip-shaped member (conjugate pad) 2 (which is impregnated with the detection antibody) and a sample addition member (sample pad) 3 arranged at one end of the lengthwise direction of the strip (upstream of the sample flow B) on the insoluble membrane carrier 1; and a member for absorption (absorption pad) 4 arranged at the other end (downstream side of the specimen flow B) at the other end of the lengthwise direction of the strip (downstream of the sample flow B) on the insoluble membrane carrier 1. Arranged in the center of the strip lengthwise direction on the insoluble membrane carrier 1 is a site 5 on which the capture antibody is immobilized, along with, if necessary, a site 6 on which the control reagent is immobilized. The control reagent is a reagent that does not bind to the analyte but does bind to the detection antibody. When a sample A is applied on the sample addition member (sample pad) 3, the sample A passes through the member (conjugate pad) 2 impregnated with the detection antibody, and flows through the insoluble membrane carrier 1 in the sample flow direction A. During this process, the analyte in the sample (in the case of the present invention, bacteria to be detected) binds to the detection antibody to form an analyte-detection antibody complex. When the sample A passes through the capture-antibody immobilized site 5, the analyte in the sample binds to the capture antibody to form a capture antibody-analyte-detection antibody complex. When the sample A passes through the control-reagent immobilized site 6, the detection antibody that has not bound to the analyte binds to the control reagent 6, whereby it is confirmed that the test has been completed (i.e., that specimen A has passed through the capture antibody 5). The presence or amount of the analyte can be detected by detecting the label of the detection antibody in the capture antibody-analyte-detection antibody complex that exists in the capture-antibody immobilized site 5 by known means. If necessary, the label of the detection antibody may be sensitized by known methods to facilitate detection.

The types of the solid-phase carrier used for the capture antibody are not restricted, but specific examples include: porous membranes made of cellulose, nitrocellulose, cellulose acetate, nylon, PVDF (polyvinylidene difluoride), glass fiber, etc.; flow channels made of glass, plastic, PDMS (poly(dimethylsiloxane)), silicone, etc.; thread, paper, fiber, etc.

The method for attaching an antibody to the solid-phase carrier is also not restricted, but specific examples include fixation via physisorption using the hydrophobicity of the antibody and fixation via chemical bonding using a functional group of the antibody.

The type of detection label used for the detection antibody is not restricted and may be selected as appropriate in accordance with the detection method. Specific examples include: metal colloids such as gold colloids, platinum colloids, and palladium colloids; non-metal colloids such as selenium colloids, alumina colloids, and silica colloids; insoluble granular materials such as colored resin particles, dye colloids, colored liposomes, etc.; enzymes that catalyze chromogenic reactions such as alkaline phosphatase, peroxidase, luciferase, etc.; fluorescent dyes, radioisotopes; and chemiluminescent labels, bioluminescent labels, electrochemiluminescent labels, etc.

The method for attaching the label to the antibody is also not restricted, but specific examples of methods that can be used include physical adsorption using the hydrophobicity of the antibody, chemisorption using a functional group of the antibody, etc.

The detection antibody-impregnated member (conjugate pad) 2 and the sample addition member (sample pad) 3 may be optionally omitted. When the present mechanism lacks the detection antibody-impregnated member (conjugate pad) 2, the same test as above can be performed by applying the sample A and the detection antibody to one end on the insoluble membrane carrier 1 in a pre-mixed state or separately, either simultaneously or sequentially.

Even when the capture antibody and the detection antibody are interchanged, a detection kit can be constructed to enable the same detection. That is, the capture antibody may be the generic antibody and the detection antibody may be the specific antibody. Alternatively, the detection antibody may be the generic antibody and the capture antibody may be the specific antibody. In either case, either or each of the generic antibody and the specific antibody may be the antibody as described above

However, as mentioned above, it is more preferable to use the generic antibody as the detection antibody. As an example, when gold colloids are used as a label for detection, excessive optimization of labeling conditions (pH, salt concentration, blocking agent, buffer type, dispersion solution type, centrifugation conditions, etc.) is necessary depending on the antibody species used. On the other hand, since the capture antibody can be simply applied and dried on the insoluble membrane carrier, there is no need to optimize conditions excessively according to the antibody species. Since the generic antibody causes antigen-antibody reactions widely regardless of bacterial species, the same generic antibody can be used (as the detection antibody) even if the bacteria to be detected are changed, thereby eliminating the need for such excessive optimization of labeling conditions. This provides a highly advantageous effect when producing an immunochromatography kit.

According to the immunochromatographic detection method and device of the lateral-flow type mentioned above, the two or more genera of bacteria as the target for detection can be detected at once on a single detection line (in the example shown in Figure 1, the capture-antibody immobilized site 5). Specifically, in the case of the conventional immunochromatographic detection method and device of the lateral-flow type, when two or more detection targets are detected, a detection line (in the example shown in Figure 1, the capture-antibody immobilized site 5) must be provided for each detection target, so that detection lines corresponding to the number of detection targets must be formed. This has resulted in the enlargement of the device, and when there are too many targets to be detected, it is difficult or impossible to detect them with a single device. On the other hand, in the case of the immunochromatographic detection method and device of the lateral-flow type, it is possible to simultaneously detect plural genera of bacteria on a single detection line, by combining the detection antibody and the capture antibody as appropriate. This enables downsizing of the device, and even when many species and genera of bacteria have to be detected, detection can be made with a single device, by determining the total amount of the bacteria to be detected on a single detection line

The present invention also provides a method for producing the above-mentioned immunochromatography kit. The method includes at least the steps of stacking the conjugate pad, to which the detection antibody is attached, on the insoluble membrane carrier, and immobilizing the capture antibody in the chromatographic development direction opposite to the conjugate pad on the insoluble membrane carrier.

Either the capture antibody or the detection antibody may be used as the specific antibody, and either may be used as the generic antibody. However, it is preferable to use the specific antibody as the capture antibody and the generic antibody as the detection antibody.

The specific antibody may preferably be an antibody that causes antigen-antibody reactions specifically with at least bacteria of one or more genera selected from the genus Escherichia, the genus Staphylococcus, the genus Pseudomonas, the genus Bacillus, the genus Klebsiella, the genus Serratia, the genus Rahnella, the genus Citrobacter, the genus Listeria, the genus Enterobacter, and the genus Salmonella.

The generic antibody may preferably be an antibody that causes antigen-antibody reactions specifically with at least bacteria of at least 5 or more genera selected from the genus Escherichia, the genus Staphylococcus, the genus Pseudomonas, the genus Bacillus, the genus Klebsiella, the genus Serratia, the genus Rahnella, the genus Citrobacter, the genus Listeria, the genus Enterobacter, and the genus Salmonella.

According to the method for producing an immunochromatography kit, it is possible to use the same generic antibody as the detection antibody for mass production of plural variations of immunochromatography kits, which is extremely effective in that plural types of immunochromatography kits can be manufactured by selecting only the capture antibody.

### EXAMPLES

The present invention will be described in more detail with reference to the examples below. However, the present invention is not bound by the following examples, and can be implemented in any form within the claimed scope.

### [1. Production of Antibodies]

### *Production of Generic Antibody A (PA51B2):

Pseudomonas aeruginosa (PA) was used as an immunogen bacterium. An antibody against ribosome protein L7/L12 of Pseudomonas aeruginosa was produced by referring to the method described in WO2000/06603 A. Specifically, E. coli transformed with an expression vector incorporating DNA encoding the entire amino acid sequence of ribosomal protein L7/L12 of Pseudomonas aeruginosa was cultured using, e.g., LB medium, and the ribosomal protein L7/L12 was purified by affinity column as a fusion protein using a tag sequence derived from the expression vector. The total length protein of Pseudomonas aeruginosa L7/L12 was prepared as an immunogen in PBS according to the standard method for obtaining hybridomas, so that the concentration of the immunogen was 0.4 mg/mL, and Freund's adjuvant was added in the same volume. Mice were immunized four times with an immunogen level of 50 µg/time. After confirming the increase in serum antibody titer by test blood collection, mouse spleen cells were harvested. The excised mouse spleen cells were fused with myeloma cells, and various hybridomas were obtained.

The various hybridomas obtained were cultured in HAT medium, and screened based on antibodies in the culture supernatant. The screening was performed by ELISA using solid-phase lysates of several genera of as antigens, to select hybridomas producing antibodies simultaneously reactive with lysates of eleven species of bacteria: Escherichia coli (EC), Staphylococcus aureus (SA), Pseudomonas aeruginosa (PA), Bacillus subtilis (BS), Klebsiella pneumoniae (KP), Serratia liquefaciens (SL), Ranella aquatilis (RA), Citrobacter freundii (CF), Listeria monocytogenes (LM), Enterobacter cloacae (ECL), and Salmonella enterica (SE). According to the established method of monoclonal antibody production, the selected hybridomas were cultured in TIL MediaI medium supplemented with 10% fetal bovine serum (FBS), injected into the abdominal cavity of a mouse, and ascites was collected. An antibody was purified from the collected ascites by centrifuging the ascites to remove suspended solids and erythrocytes, filtering the supernatant through a filter with a mesh size of 0.45 µm, and pass the filtrate through a Protein G column to adsorb the antibody, to thereby produce a generic antibody A (PA51B2), which corresponds to an antibody as recited in the present invention.

### *Production of Generic Antibody B (LP54A3)

The same procedure as that for producing the generic antibody A was carried out except that ribosome protein L7/L12 of Legionella pneumophilia (LP) was used as an immunogen, to thereby produce a generic antibody B (LP54A3), which corresponds to an antibody as recited in the present invention.

### *Production of Generic Antibody C (CP141A190.1)

The same procedure as that for producing the generic antibody A was carried out except that ribosome protein L7/L12 of Chlamydia pneumoniae (CP) was used as an immunogen, to thereby produce a generic antibody C (CP141A190.1), which corresponds to an antibody as recited in the present invention.

### *Production of Specific Antibody A (HI142D11.3)

Haemophilus influenzae (HI) was used as an immunogen bacterium. An antibody against ribosome protein L7/L12 of Haemophilus influenzae was produced by referring to the method described in WO2000/06603 A. Specifically, E. coli transformed with an expression vector incorporating DNA encoding the entire amino acid sequence of ribosomal protein L7/L12 of Haemophilus influenzae was cultured using, e.g., LB medium, and the ribosomal protein L7/L12 was purified by affinity column as a fusion protein using a tag sequence derived from the expression vector. The total length protein of Haemophilus influenzae L7/L12 was prepared as an immunogen in PBS according to the standard method for obtaining hybridomas, so that the concentration of the immunogen was 0.4 mg/mL, and Freund's adjuvant was added in the same volume. Mice were immunized four times with an immunogen level of 50 µg/time. After confirming the increase in serum antibody titer by test blood collection, mouse spleen cells were harvested. The excised mouse spleen cells were fused with myeloma cells, and various hybridomas were obtained.

The various hybridomas obtained were cultured in HAT medium, and screened based on antibodies in the culture supernatant. The screening was performed by ELISA using solid-phase lysates of several genera of bacteria as antigens, to select hybridomas producing antibodies simultaneously reactive with lysates of nine species of bacteria: Escherichia coli (EC), Pseudomonas aeruginosa (PA), Klebsiella pneumoniae (KP), Serratia liquefaciens (SL), Rahnella aquatilis (RA), Citrobacter freundii (CF), Listeria monocytogenes (LM), Enterobacter cloacae (ECL), and Salmonella enterica (SE). According to the established method of monoclonal antibody production, the selected hybridomas were cultured in TIL MediaI medium supplemented with 10% fetal bovine serum (FBS), injected into the abdominal cavity of a mouse, and ascites was collected. An antibody was purified from the collected ascites by centrifuging the ascites to remove suspended solids and erythrocytes, filtering the supernatant through a filter with a mesh size of 0.45 µm, and pass the filtrate through a Protein G column to adsorb the antibody, to thereby produce a specific antibody A(HI142D11.3).

### *Production of Specific Antibody B (SA75B2)

Various hybridomas were obtained by the same procedure as described above using Staphylococcus aureus (SA) as the immunogen bacteria, and hybridomas that produced antibodies reactive with bacterial lysates of the two bacteria (Staphylococcus aureus (SA) and Bacillus subtilis (BS)) were selected. A specific antibody B (SA75B2) was then produced by the same procedure as described above.

### *Production of Specific Antibody C (PA78A2)

Various hybridomas were obtained by the same procedure as that for producing the generic antibody A, except that Pseudomonas aeruginosa (PA) was used as the immunogen bacteria and ribosomal protein L7/L12 of Pseudomonas aeruginosa was used as the immunogen. Hybridomas producing antibodies reactive with bacterial lysate of one species (Pseudomonas aeruginosa (PA)) were selected. A specific antibody C (PA78A2) was then produced using the same procedure as described above.

### *Production of Specific Antibody D (EC50C1)

Various hybridomas were obtained by the same procedure as that for producing the generic antibody A, except that E. coli (EC) was used as the immunogen bacteria and ribosomal protein L7/L12 of E. coli was used as the immunogen. Hybridomas producing antibodies reactive with bacterial lysates of seven species (E. coli (EC), Klebsiella pneumoniae (KP), Serratia liquefaciens (SL), Rahnella aquatilis (RA), Citrobacter freundii (CF), Enterobacter cloacae (ECL), and Salmonella enterica (SE)) were selected. A specific antibody D (EC50C1) was then produced using the same procedure as described above.

### *Amino Acid Sequencing of Each Variable Region of the Heavy and Light Chains of Generic Antibodies A to C and Specific Antibodies A to D:

The amino acid sequences of each variable sequence of the heavy and light chains for the generic antibodies A to C (antibodies of the invention) and the specific antibodies A to D produced by the above procedure were determined according to the usual method. The correspondence between the amino acid sequences and the sequence identification numbers is shown below.

### *Generic Antibody A (PA51B2):

Amino acid sequence of the heavy chain variable region (SEQ ID NO:1)
Amino acid sequence of the light chain variable region (SEQ ID NO:2) *Generic Antibody B (LP54A3):
Amino acid sequence of the heavy chain variable region (SEQ ID NO:3)
Amino acid sequence of the light chain variable region (SEQ ID NO:4) *Generic Antibody C (CP141A190.1):
Amino acid sequence of the heavy chain variable region (SEQ ID NO:5)
Amino acid sequence of the light chain variable region (SEQ ID NO:6) Specific Antibody A (HI142D11.3):
Amino acid sequence of the heavy chain variable region (SEQ ID NO:7)
Amino acid sequence of the light chain variable region (SEQ ID NO:8) *Specific Antibody B (SA75B2):
Amino acid sequence of the heavy chain variable region (SEQ ID NO:9)
Amino acid sequence of the light chain variable region (SEQ ID NO:10) *Specific Antibody C (PA78A2):
Amino acid sequence of the heavy chain variable region (SEQ ID NO:11)
Amino acid sequence of the light chain variable region (SEQ ID NO:12) *Specific Antibody D (EC50C1):
Amino acid sequence of the heavy chain variable region (SEQ ID NO:13)
Amino acid sequence of the light chain variable region (SEQ ID NO:14)

### [2. Detection of Antigen-Antibody Reactions between Antibodies and Bacteria 1 - Lysis Antigen Solid-Phase ELISA]

The thus-produced Generic Antibodies A to C (antibodies of the present invention) and Specific Antibodies A to D were used to obtain data on the reactivity with plural genera of bacteria by means of ELISA. Eleven species of bacteria were selected for the study: Escherichia coli (EC), Staphylococcus aureus (SA), Pseudomonas aeruginosa (PA), Bacillus subtilis (BS), Klebsiella pneumoniae (KP), Serratia liquefaciens (SL), Rahnella aquatilis (RA), Citrobacter freundii (CF), Listeria monocytogenes (LM), Enterobacter cloacae (ECL), and Salmonella enterica (SE), which are frequently detected in food, beverage, environmental, or biological samples. The eleven species of bacteria described above were purchased and cultured from ATCC, prepared at 1 x e⁸ cfu/mL each, and suspended in PBS. Each bacterium was lysed by sonication, and debris was removed by filtration through a filter with a 0.45-µm mesh opening to obtain the bacterial lysate of each bacterium.

50 µL of the above bacterial lysate was dropped into each well of a 96-well polystyrene plate for ELISA and solidified on the bottom of the plate. After washing each well three times with PBS-T (PBS with Tween 20), blocking treatment was performed with PBS containing 1% BSA (Bovine Serum Albumin). After blocking, the wells were washed three times with PBS-T, and 50 µL of 10 µg/mL solution of each of the Generic Antibodies A to C and Specific Antibodies A to D was dropped into each well for 1 hour for antigen-antibody reactions. After the reactions, the samples were washed three times with PBS-T, and then 0.5 µg/mL of HRP (Horse Radish Peroxidase) labeled secondary antibody (Goat Anti-mouse IgG: Goat Anti-mouse IgG), which is the enzyme for detection, was added dropwise. After the reaction, the wells were washed five times with PBS-T, and 100 µL of a mixture of tetramethylbenzidine (TMB) and hydrogen peroxide, the chromogenic substrate, was added dropwise to each well for the chromogenic reaction. After 10 minutes, hydrochloric acid, the reaction stopping solution, was dropped into each well, and the absorbance of each well at 450 nm was measured with a plate reader.

The measurement results are shown in Table 1 below. The results shown in this table indicate that the Generic Antibodies A to C reacted more sensitively (with an absorbance of 0.3 or higher for all bacteria, and 1.0 or higher in most cases) with the samples of the above-mentioned bacteria, compared to the sample without bacteria. In other words, it was confirmed that the Generic Antibodies A to C, which were obtained via immunization with different bacteria, can cause antigen-antibody reactions (detectable) with all of the bacterial samples mentioned above. On the other hand, Specific Antibodies A to D showed antigen-antibody reactivity only with specific ones of these bacteria.

### [Table 1]

**Table 1**

| | Negative Control | Bacteria | | | | | | | | | | |
|---|---|---|---|---|---|---|---|---|---|---|---|---|
| | Without Bacteria | EC | SA | PA | BS | KP | SL | RA | CF | LM | ECL | SE |
| Generic Antibody A | - | + | + | + | + | + | + | + | + | + | + | + |
| Generic Antibody B | - | + | + | + | + | + | + | + | + | + | + | + |
| Generic Antibody C | - | + | + | + | + | + | + | + | + | + | + | + |
| Specific Antibody A | - | + | - | + | - | + | + | + | + | + | + | + |
| Specific Antibody B | - | - | + | - | + | - | - | - | - | - | - | - |
| Specific Antibody C | - | - | - | + | - | - | - | - | - | - | - | - |
| Specific Antibody D | - | + | - | - | - | + | + | + | + | - | + | + |

| | | | | | | | | | | | | |
|---|---|---|---|---|---|---|---|---|---|---|---|---|
| -: Absorbance < 0.3 +: Absorbance ≥ 0.3 | | | | | | | | | | | | |

### [3. Detection of Antigen-Antibody Reactions between Antibodies and 2 - Recombinant Antigen Solid-Phase ELISA]

The thus-produced Generic Antibodies A to C (antibodies of the present invention) and Specific Antibodies A to D were used to obtain data on the reactivity with ribosome protein L7/L12 of plural genera of bacteria by means of ELISA. Eleven species of bacteria were selected for the study: Escherichia coli (EC), Staphylococcus aureus (SA), Pseudomonas aeruginosa (PA), Bacillus subtilis (BS), Klebsiella pneumoniae (KP), Serratia liquefaciens (SL), Rahnella aquatilis (RA), Citrobacter freundii (CF), Listeria monocytogenes (LM), Enterobacter cloacae (ECL), and Salmonella enterica (SE), which are frequently detected in food, beverage, environmental, or biological samples. E. coli transformed with expression vectors incorporating DNA encoding the amino acid sequence of ribosomal protein L7/L12 of each of the above eleven species of bacteria were cultured using, e.g., LB medium, and the ribosomal protein L7/L12 was purified as a fusion protein by affinity columns using tag sequences derived from the expression vector.

50 µL of 10 ng/mL solution of the ribosomal protein L7/L12 of each bacterial species was dropped into each well of a 96-well polystyrene plate for ELISA and solidified on the bottom of the plate. After washing each well three times with PBS-T (PBS with Tween 20), blocking treatment was performed with PBS containing 1% BSA (Bovine Serum Albumin). After blocking, the wells were washed three times with PBS-T, and 50 µL of 10 µg/mL solution of each of the Generic Antibodies A to C and Specific Antibodies A to D was dropped into each well for 1 hour for antigen-antibody reactions. After the reactions, the samples were washed three times with PBS-T, and then 0.5 µg/mL of HRP (Horse Radish Peroxidase) labeled secondary antibody (Goat Anti-mouse IgG: Goat Anti-mouse IgG), which is the enzyme for detection, was added dropwise. After the reaction, the wells were washed five times with PBS-T, and 100 µL of a mixture of tetramethylbenzidine (TMB) and hydrogen peroxide, the chromogenic substrate, was added dropwise to each well for the chromogenic reaction. After 10 minutes, hydrochloric acid, the reaction stopping solution, was dropped into each well, and the absorbance of each well at 450 nm was measured with a plate reader.

The measurement results are shown in Table 2 below. The results shown in this table indicate that the Generic Antibodies A to C reacted more sensitively (with an absorbance of 0.3 or higher for all bacteria, and 1.0 or higher in most cases) with the ribosomal protein L7/L12 samples of the above-mentioned bacteria, compared to the sample without bacteria. In other words, it was confirmed that the Generic Antibodies A to C, which were obtained via immunization with different bacteria, can cause antigen-antibody reactions (detectable) with all of the bacterial samples mentioned above. On the other hand, Specific Antibodies A to D showed antigen-antibody reactivity only with specific ones of these bacteria.

### [Table 2]

**Table 2**

| | Negative Control | Bacteria | | | | | | | | | | |
|---|---|---|---|---|---|---|---|---|---|---|---|---|
| | Without Bacteria | EC | SA | PA | BS | KP | SL | RA | CF | LM | ECL | SE |
| Generic Antibody A | - | + | + | + | + | + | + | + | + | + | + | + |
| Generic Antibody B | - | + | + | + | + | + | + | + | + | + | + | + |
| Generic Antibody C | - | + | + | + | + | + | + | + | + | + | + | + |
| Specific Antibody A | - | + | - | + | - | + | + | + | + | + | + | + |
| Specific Antibody B | - | - | + | - | + | - | - | - | - | - | - | - |
| Specific Antibody C | - | - | - | + | - | - | - | - | - | - | - | - |
| Specific Antibody D | - | + | - | - | - | + | + | + | + | - | + | + |

| | | | | | | | | | | | | |
|---|---|---|---|---|---|---|---|---|---|---|---|---|
| -: Absorbance < 0.3 +: Absorbance ≥ 0.3 | | | | | | | | | | | | |

### [4. Verification of Cross-Reactivity between Antibodies and Non-Bacterial Components in Food/Beverage/Environmental Sample]

The thus-produced Generic Antibodies A to C (antibodies of the present invention) and Specific Antibodies A to D were used to obtain data on the reactivity with non-bacterial components in various food, beverage, or environmental samples (food and environmental components) by means of ELISA. Raw fish (yellowtail and horse mackerel), raw noodles (yakisoba), raw egg, prepared food (potato salad), vegetables (cucumber (fruit vegetable), carrot (root vegetable), and lettuce (leaf vegetable)), and meats and processed meats (beef ribs, beef short ribs, pork loin, chicken breast, and ham) were purchased at a supermarket and used as food samples. Each of these foodstuffs was weighed 25 g, placed in a commercial stomacher bag, and stomached with 225 ml of PBS. A portion of the stomacher-treated solution was processed through a filter with a mesh aperture of 0.45 µm to remove solids containing bacteria, thereby preparing a bacteria-free food sample for ELISA. Milk and tea were purchased at a supermarket and used as beverage samples. Each of these beverages was suspended in PBS to a concentration of 1/10, and solids containing bacteria were removed with a filter with a 0.45 µm mesh opening, thereby preparing a bacteria-free beverage sample for ELISA. As environmental samples, hand fingers, cutting boards, kitchen knives, and refrigerator handles were wiped with a commercially available wiping kit (ELMEX Pro-mediaST-25, PBS), suspended in PBS provided with the kit, and solids containing bacteria were removed with a filter with a 0.45 µm mesh opening, thereby preparing a bacteria-free environmental sample for ELISA.

50 µL of each of the food, beverage, and environment samples was dropped into each well of a 96-well polystyrene plate for ELISA and solidified on the bottom of the plate. After washing each well three times with PBS-T (PBS with Tween 20), blocking treatment was performed with PBS containing 1% BSA. After blocking, the wells were washed three times with PBS-T, and 50 µL of 10 µg/mL solution of each of the Generic Antibodies A to C and Specific Antibodies A to D was dropped into each well for 1 hour for reactions. After the reactions, the samples were washed three times with PBS-T, and then 0.5 µg/mL of HRP (Horse Radish Peroxidase) labeled secondary antibody (Goat Anti-mouse IgG: Goat Anti-mouse IgG), which is the enzyme for detection, was added dropwise. After the reaction, the wells were washed five times with PBS-T, and 100 µL of a mixture of TMB and hydrogen peroxide, the chromogenic substrate, was added dropwise to each well for the chromogenic reaction. After 10 minutes, hydrochloric acid, the reaction stopping solution, was dropped into each well, and the absorbance of each well at 450 nm was measured with a plate reader.

The measurement results are shown in Table 3 below. The results shown in this table indicate that the Generic Antibodies A to C did not react with any of the above non-bacterial components in the food, beverage, or environmental samples (with an absorbance of less than 0.3 for each sample). Combined with the results of the aforementioned examples, it was confirmed that all of the Generic Antibodies A to C do not react with any of the non-bacterial components in the food, beverage, or environmental samples (food, beverage, or environmental components), but only with the specific plural genera of bacteria to be detected. In other words, it was confirmed that these antibodies can detect with high selectivity the presence and/or amount of the specific plural genera of bacteria to be detected in the food, beverage, or environmental samples. On the other hand, the Specific Antibodies A to D did not show cross-reactivity with any of the non-bacterial components in the food, beverage, or environmental samples.

**[Table 3-1]**

| Table3 | | | | | | | |
|---|---|---|---|---|---|---|---|
| | Food or beverage samples | | | | | | |
| | Raw fish | Raw noodles | Raw egg | Prepared food | Vegetables | Meat | Beverage |
| Generic Antibody A | - | - | - | - | - | - | - |
| Generic Antibody B | - | - | - | - | - | - | - |
| Generic Antibody C | - | - | - | - | - | - | - |
| Specific Antibody A | - | - | - | - | - | - | - |
| Specific Antibody B | - | - | - | - | - | - | - |
| Specific Antibody C | - | - | - | - | - | - | - |
| Specific Antibody D | - | - | - | - | - | - | - |

| | | | | | | | |
|---|---|---|---|---|---|---|---|
| -: Absorbance < 0.3 +: Absorbance ≧ 0.3 | | | | | | | |

**[Table 3-2]**

| Table3 (continued) | | | | |
|---|---|---|---|---|
| | Environmental samples | | | |
| | Hand fingers | Cutting board | Kitchen knife | Refrigerator handle |
| Generic Antibody A | - | - | - | - |
| Generic Antibody B | - | - | - | - |
| Generic Antibody C | - | - | - | - |
| Specific Antibody A | - | - | - | - |
| Specific Antibody B | - | - | - | - |
| Specific Antibody C | - | - | - | - |
| Specific Antibody D | - | - | - | - |

| | | | | |
|---|---|---|---|---|
| -: Absorbance < 0.3 +: Absorbance ≧ 0.3 | | | | |

### [5. Production of Immunochromatographic Detection Kits]

### * Production of Immunochromatographic Detection Kits:

Three types of immunochromatographic detection kits (a) to (c) were prepared by using the Generic Antibodies A to C (antibodies of the present invention) as secondary antibodies (detection antibodies) and the Specific Antibodies A to D as primary antibodies (capture antibodies) in the combinations shown in (a) to (c) of Table 4 below. In addition, additional three types of immunochromatographic detection kits (d) to (f) capable of similar detection were prepared by changing the antibodies used for the primary and secondary antibodies in the combinations listed in (d) to (f) of Table 4 below. It was possible to construct various detection kits easily without excessive optimization of labeling conditions for each antibody, by using the common Generic Antibody A as a detection antibody.

### [Table 4]

**Table 4**

| Immunochromatography detection kit | Bacteria to be detected | Primary antibody (capture antibody) | Secondary antibody (detection antibody) |
|---|---|---|---|
| (a) | Pseudomonas aeruginosa (PA) | Specific Antibody C (PA78A2) | Generic Antibody A (PA51B2) |
| (b) | Enterobacteriaceae bacteria | Specific Antibody D (EC50C1) | Generic Antibody A (PA51B2) |
| (c) | All bacteria | Specific Antibody A (HI142D11.3) & Specific Antibody B (SA75B2) | Generic Antibody A (PA51B2) |
| (d) | Pseudomonas aeruginosa (PA) | Generic Antibody A (PA51B2) | Specific Antibody C (PA78A2) |
| (e) | Enterobacteriaceae bacteria | Generic Antibody A (PA51B2) | Specific Antibody D (EC50C1) |
| (f) | All bacteria | Generic Antibody A (PA51B2) | Specific Antibody A (HI142D11.3) & Specific Antibody B (SA75B2) |

### *Production of Membrane Carrier for Immunochromatography Development:

A solution was prepared that contain 1.5 mg/mL of each primary antibody (the Specific Antibody C for (a), the Specific Antibody D for (b), and a mixture of the Specific Antibodies A and B for (c)) and 3% (v/v) of trehalose in 10 mM sodium phosphate buffer solution. The resulting solution was applied to a commercially available nitrocellulose membrane cut to 2.5 cm wide and 15 cm long at a volume of 1 µL solution per cm² and dried to make a membrane carrier for immunochromatography development.

### *Preparation of Antibodies for Gold Colloid Labeling Detection and Antibody-Impregnated Members for Gold Colloid Labeling Detection:

A commercially available gold colloid solution (60 nm particle size) was added to the tube, and then 100 mM potassium phosphate buffer (pH 6.5) was added and mixed. Here, 1/10 volume of secondary antibody (Generic Antibody A) was added and mixed to prepare a solution with an antibody concentration of 0.1 mg/mL. The solution was allowed to stand at room temperature for 30 minutes to allow the antibody to bind to the surface of the gold colloidal particles. Then, casein solution was added so that the final concentration in the gold colloid solution was 0.1%, mixed, and allowed to stand for 60 minutes for blocking treatment. The solution was centrifuged at 8000g for 20 minutes, and the supernatant was removed with a pipette. The gold colloid dispersion solution (0.25% casein, 40 mM NaCl, 5% sucrose, 10 mM Tris-HCl (pH 8.2)) was added, mixed, and redispersed to prepare a gold colloid-labeled antibody solution for detection. This detection antibody solution was soaked into commercially available glass fiber sheets and dried to prepare an antibody-impregnated member for gold colloid labeling detection.

### *Assembly of Immunochromatographic Detection Kits:

In addition to the membrane carrier for immunochromatographic development and the antibody-impregnated member for gold colloid labeling detection prepared by the procedures described above, a cotton cloth for adding a sample and a filter paper for absorbing a sample were also prepared for each kit. These components were laminated onto a commercially available polyethylene substrate, cut into 5 mm widths, to prepare an immunochromatographic detection kit with a detection mechanism as shown in Figure 1.

### [6. Detection of Bacteria using the Immunochromatographic Detection Kits]

The thus-obtained immunochromatographic detection kits (a) to (c) were used to detect plural genera of bacteria. Eleven species of bacteria were selected for the study: Escherichia coli (EC), Staphylococcus aureus (SA), Pseudomonas aeruginosa (PA), Bacillus subtilis (BS), Klebsiella pneumoniae (KP), Serratia liquefaciens (SL), Rahnella aquatilis (RA), Citrobacter freundii (CF), Listeria monocytogenes (LM), Enterobacter cloacae (ECL), and Salmonella enterica (SE), which are frequently detected in food, beverage, environmental, or biological samples. The eleven species of bacteria were each prepared at 1 × e⁸ cfu/mL and suspended in PBS. Each bacterium was lysed by sonication to obtain bacterial lysates (bacterial samples for immunochromatographic assay) of the eleven species of bacteria. A PBS solution without bacterial lysates was also prepared as a bacteria-free sample. Tween 20 was added to each of these samples at a final concentration of 1%, whereby samples for immunochromatographic development were prepared.

The prepared samples (one bacteria-free sample and 11 bacterial lysate samples) were each added to the sample addition member area of each of the immunochromatographic detection kits mentioned above, and 30 minutes later, the line coloration of the capture-antibody applied area of the membrane carrier was visually confirmed.

The prepared samples (one bacteria-free sample and 11 bacterial lysate samples) were each added to the sample addition member area of each of the immunochromatographic detection kits having the configurations of (a) through (c) above, and 30 minutes later, the line coloration of the capture-antibody applied area of the membrane carrier was visually confirmed. The results are shown in Table 5 below. These results indicate that as intended, only one species, Pseudomonas aeruginosa (PA), was detected with the detection kit (a), seven species belonging to Enterobacteriaceae bacteria were detected with the detection kit (b), and all 11 species (all bacteria) were detected with the detection kit (c). In other words, each immunochromatographic detection kit constructed by combining a specific antibody that causes antigen-antibody reactions only with bacteria of limited genera (i.e., Specific antibody A (PA78A2), Specific Antibody B (EC50C1), Specific Antibody C (HI142D11.3), and/or Specific Antibody D (SA75B2) described above) and a generic antibody that causes antigen-antibody reactions widely with bacteria of many genera (i.e., Generic Antibody A (PA51B2) described above) was shown to be able to detect the target bacteria easily and rapidly.

As mentioned above, even if the species of bacteria to be detected are changed, it is possible to use a common generic antibody (which may preferably be used as a detection antibody) and combine it with a specific antibody (which may preferably be used as a capture antibody) in accordance with the species of bacteria to be detected, thereby producing an immunochromatographic detection kit that can detect the target species of bacteria without excessive examination of conditions. The detection kits using combinations of (d) through (f) in Table 4 above were also tested for detection of the plural genera of Enterobacteriaceae, and similar results were obtained.

### [Table 5]

**Table 5**

| Detection kit | Negative control | Bacteria | | | | | | | | | | |
|---|---|---|---|---|---|---|---|---|---|---|---|---|
| No. | Without bacteria | EC | SA | PA | BS | KP | SL | RA | CF | LM | ECL | SE |
| (a) | - | - | - | + | - | - | - | - | - | - | - | - |
| (b) | - | + | - | - | - | + | + | + | + | - | + | + |
| (c) | - | + | + | + | + | + | + | + | + | + | + | + |

| | | | | | | | | | | | | |
|---|---|---|---|---|---|---|---|---|---|---|---|---|
| -: Visually negative +: Visually positive | | | | | | | | | | | | |

### [7. Verification of Cross-Reactivity between Antibodies and Components in Food/Beverage/Environmental Samples using Immunochromatographic Detection Kit]

The thus-obtained immunochromatographic detection kits (a) to (c) were used to obtain data on the reactivity with non-bacterial components in various food, beverage, or environmental samples (food, beverage, or environmental components). Raw fish (yellowtail and horse mackerel), raw noodles (yakisoba), raw egg, prepared food (potato salad), vegetables (cucumber (fruit vegetable), carrot (root vegetable), and lettuce (leaf vegetable)), and meats and processed meats (beef ribs, beef short ribs, pork loin, chicken breast, and ham) were purchased at a supermarket and used as food samples. Each of these foodstuffs was weighed 25 g, placed in a commercial stomacher bag, and stomached with 225 ml of PBS. A portion of the stomacher-treated solution was processed through a filter with a mesh aperture of 0.45 µm to remove solids containing bacteria, thereby preparing a sample for immunochromatography. Milk and tea were purchased at a supermarket and used as beverage samples. Each of these beverages was suspended in PBS to a concentration of 1/10, and solids containing bacteria were removed with a filter with a 0.45 µm mesh opening, thereby preparing a sample for immunochromatography. As environmental samples, hand fingers, cutting boards, kitchen knives, and refrigerator handles were wiped with a commercially available wiping kit (ELMEX Pro-mediaST-25, PBS), suspended in PBS provided with the kit, and solids containing bacteria were removed with a filter with a 0.45 µm mesh opening, thereby preparing a sample for immunochromatography.

The samples prepared for immunochromatography from various food, beverage, or environmental samples were each added to the sample addition member area of each of the immunochromatographic detection kits having the configurations of (a) through (c) above, and 30 minutes later, the line coloration of the capture-antibody applied area of the membrane carrier was visually confirmed. The results for the detection kits (a) through (c) are shown in Table 6. As intended, none of the detection kits (a) through (c) showed cross-reactivity with the components in the above food, beverage, or environmental samples. In combination with the aforementioned examples, it was confirmed that the immunochromatographic detection kits (a) through (c) prepared by combining the aforementioned generic and specific antibodies do not react with non-bacterial components (food, beverage, or environmental components) in the food, beverage, or environmental samples, and makes it possible to detect only the plural species of bacteria to be detected easily and rapidly with high selectivity. The detection kits using combinations of (d) through (f) in Table 4 above were also tested for cross-reactivity with non-bacterial components in the food, beverage, or environmental samples, and similar results were obtained.

**[Table 6-1]**

| Table 6 | | | | | | | |
|---|---|---|---|---|---|---|---|
| Detection kit | Food or beverage samples | | | | | | |
| No. | Raw fish | Raw noodles | Raw egg | Prepared food | Vegetables | Meat | Beverage |
| (a) | - | - | - | - | - | - | - |
| (b) | - | - | - | - | - | - | - |
| (c) | - | - | - | - | - | - | - |

| | | | | | | | |
|---|---|---|---|---|---|---|---|
| -: Visually negative +: Visually positive | | | | | | | |

**[Table 6-2]**

| Table 6 (continued) | | | | |
|---|---|---|---|---|
| Detection kit | Environmental samples | | | |
| No. | Hand fingers | Cutting board | Kitchen knife | Refrigerator handle |
| (a) | - | - | - | - |
| (b) | - | - | - | - |
| (c) | - | - | - | - |

| | | | | |
|---|---|---|---|---|
| -: Visually negative +: Visually positive | | | | |

### INDUSTRIAL APPLICABILITY

The present invention can be widely used in fields where simultaneous and simple detection of plural genera of bacteria in food, beverage, environmental, or biological samples is required, mainly in the fields of medicines and foods, and therefore has extremely high industrial usefulness.

### EXPLANATION OF SYMBOLS

- 10: Detection mechanism
- 1: Insoluble membrane carrier for chromatography development
- 2: Detection antibody-impregnated member (conjugate pad)
- 3: Sample addition member (sample pad)
- 4: Member for absorption (absorption pad)
- 5: Capture-antibody immobilized site
- 6: Control-reagent immobilized site
- A: Sample
- B: Sample flow

## Claims

1. A method for detecting the presence and/or amount of bacteria in a sample selected from food or beverage samples, environmental samples, and biological samples, the method comprising the step of simultaneously detecting the presence and/or amount of at least bacteria of two or more different genera in the sample based on antigen-antibody reactions,
wherein the step of detecting comprises: contacting the sample with an antibody that causes antigen-antibody reactions with components derived from the bacteria of two or more genera; and measuring the presence and/or intensity of the antigen-antibody reaction that occurs in the sample after contact,
wherein the antibody is a monoclonal antibody having:
as a heavy chain variable region sequence, the amino acid sequence having the amino acid sequence of SEQ ID NO:1, and as a light chain variable region sequence, the amino acid sequence having the amino acid sequence of SEQ ID NO:2; or
as a heavy chain variable region sequence, the amino acid sequence having the amino acid sequence of SEQ ID NO:3, and as a light chain variable region sequence, the amino acid sequence having the amino acid sequence of SEQ ID NO:4; or
as a heavy chain variable region sequence, the amino acid sequence having the amino acid sequence of SEQ ID NO:5, and as a light chain variable region sequence, the amino acid sequence having the amino acid sequence of SEQ ID NO:6.

2. The method according to claim 1, wherein the step of detecting includes simultaneously detecting bacteria of two or more, or 3 or more, or 4 or more, or 5 or more, or 6 or more, or 7 or more, or 8 or more, or 9 or more, or 10 or more, or 11 or more different genera selected from the group consisting of the genus Escherichia, the genus Staphylococcus, the genus Pseudomonas, the genus Bacillus, the genus Klebsiella, the genus Serratia, the genus Rahnella, the genus Citrobacter, the genus Listeria, the genus Enterobacter, and the genus Salmonella,
optionally wherein the bacteria of two or more genera to be detected include a Gram-negative bacterium and a Gram-positive bacterium.

3. The method according to claim 1 or 2, wherein the antibody is an antibody that causes antigen-antibody reactions with ribosome proteins L7/L12 of the bacteria of two or more genera.

4. The method according to claim 3, wherein the antibody does not cause cross-reactions with at least one non-bacterial component that may be present in the sample,
optionally wherein the non-bacterial component with which the antibody does not cause cross-reactions is an organic component derived from a virus, plant, and/or animal.

5. The method according to any one of claims 1 to 4, comprising detecting the presence and/or amount of bacteria in the sample by the steps of:
(I) capturing the bacteria in the sample and detecting the bacteria in the sample based on antigen-antibody reactions between the sample, a capture antibody bound to a solid- phase carrier, and a detection antibody having a detection label, and
(II) detecting the bacteria to be detected in the sample based on the detection label, wherein one of the capture antibody and the detection antibody is at least one specific antibody, which causes antigen-antibody reactions with one or more bacteria to be detected, and the other is at least one generic antibody, which causes antigen-antibody reactions with bacteria of five or more genera including the bacteria to be detected, and
wherein either the generic antibody or the specific antibody is an antibody as recited in any one of claims 1 to 4.

6. The method according to claim 5, wherein step (I) includes the steps of:
(Ia-1) contacting the detection antibody with the sample to thereby detect bacteria in the sample based on the antigen-antibody reaction between the detection antibody and the bacteria; and
(Ia-2) contacting the capture antibody with the sample containing the bacteria labeled by the detection antibody to thereby capture the bacteria in the sample based on the antigen- antibody reaction between the capture antibody and the bacteria-detection antibody complex, or
wherein step (I) includes the steps of:
(Ib-1) contacting the capture antibody with the sample to thereby capture bacteria in the sample based on the antigen-antibody reaction between the capture antibody and the bacteria, and
(Ib-2) contacting the detection antibody with the sample containing the bacteria captured by the capture antibody to thereby detect the bacteria in the sample based on the antigen-antibody reaction between the detection antibody and the bacteria-capture antibody complex.

7. The method according to claim 5 or 6, wherein the capture antibody is the generic antibody, and the detection antibody is the specific antibody, or
wherein the detection antibody is the generic antibody, and the capture antibody is the specific antibody.

8. The method according to any one of claims 5 to 7, wherein the generic antibody causes antigen-antibody reactions with bacteria of five or more genera selected from at least the genus Escherichia, the genus Staphylococcus, the genus Pseudomonas, the genus Bacillus, the genus Klebsiella, the genus Serratia, the genus Rahnella, the genus Citrobacter, the genus Listeria, the genus Enterobacter, and the genus Salmonella, and/or
wherein the specific antibody causes antigen-antibody reactions specifically with bacteria of one or more genera selected from at least the genus Escherichia, the genus Staphylococcus, the genus Pseudomonas , the genus Bacillus, the genus Klebsiella, the genus Serratia, the genus Rahnella, the genus Citrobacter, the genus Listeria, the genus Enterobacter, and the genus Salmonella.

9. The method according to any one of claims 5 to 8, wherein the generic antibody comprises:
as a heavy chain variable region sequence, the amino acid sequence having the amino acid sequence of SEQ ID NO: 1, and as a light chain variable region sequence, the amino acid sequence having the amino acid sequence of SEQ ID NO:2; or
as a heavy chain variable region sequence, the amino acid sequence having the amino acid sequence of SEQ ID NO:3, and as a light chain variable region sequence, the amino acid sequence having the amino acid sequence of SEQ ID NO:4; or
as a heavy chain variable region sequence, the amino acid sequence having amino acid sequence of SEQ ID NO:5, and as a light chain variable region sequence, the amino acid sequence having the amino acid sequence of SEQ ID NO:6, and/or
wherein the specific antibody comprises:
as a heavy chain variable region sequence, the amino acid sequence having the amino acid sequence of SEQ ID NO:7, and as a light chain variable region sequence, the amino acid sequence having
the amino acid sequence of SEQ ID NO:8; or
as a heavy chain variable region sequence, amino acid sequence having the amino acid sequence of SEQ ID NO:9, and as a light chain variable region sequence, the amino acid sequence having the amino acid sequence of SEQ ID NO:10; or
as a heavy chain variable region sequence, the amino acid sequence having the amino acid sequence of SEQ ID NO:11, and as a light chain variable region sequence, the amino acid sequence having the amino acid sequence of SEQ ID NO:12; or
as a heavy chain variable region sequence, the amino acid sequence having the amino acid sequence of SEQ ID NO:13, and as a light chain variable region sequence, the amino acid sequence having the amino acid sequence of SEQ ID NO:14.

10. A method for determining the degree of contamination by bacteria in a sample selected from food or beverage samples, environmental samples, and biological samples, comprising the step of simultaneously detecting the presence and/or amount of bacteria of one or two or more genera in the sample based on antigen-antibody reactions by the method according to any one of claims 1 to 9.

11. A kit for detecting the presence and/or amount of bacteria in a sample selected from food or beverage samples, environmental samples, and biological samples by the method according to any one of claims 1 to 4, comprising an antibody as recited in any one of claims 1 and 3 to 4.

12. A kit for detecting the presence and/or amount of bacteria in a sample selected from food or beverage samples, environmental samples, and biological samples by the method according to any one of claims 5 to 9, comprising a capture antibody and a detection antibody as recited in any one of claims 5 to 9, wherein one of the capture antibody and the detection antibody is the generic antibody and the other is the specific antibody,
optionally wherein the kit further comprises an insoluble membrane carrier for developing the sample and contacting the sample with the capture antibody, wherein the insoluble membrane carrier has a detection line formed thereon and the capture antibody is immobilized on the detection line, whereby two or more species of bacteria in the sample are detected on the single detection line.

13. The kit according to claim 11 or 12, which is an immunochromatography kit, optionally wherein the capture antibody and the detection antibody are selected so as to cause antigen-antibody reactions with the ribosome protein L7/L 12 of the bacteria to be detected in the sample to form a sandwich structure,
wherein the immunochromatography kit further comprises:
an insoluble membrane carrier for developing the sample and contacting the sample with the capture antibody; and
a conjugate pad which is formed on the insoluble membrane carrier and to which the detection antibody is attached, and
wherein the capture antibody is immobilized to the conjugate pad on the insoluble membrane carrier along the development direction of chromatography.

14. A method for producing an immunochromatography kit according to claim 13, comprising the steps of:
placing, on the insoluble membrane carrier, the conjugate pad to which the detection antibody is attached; and
immobilizing the capture antibody to the conjugate pad on the insoluble membrane carrier along the development direction of chromatography,
optionally wherein the specific antibody is used as the capture antibody, and the generic antibody is used as the detection antibody.

## Patentansprüche

1. Verfahren zum Nachweis des Vorhandenseins und/oder der Menge von Bakterien in einer Probe, die aus Lebensmittel- oder Getränkeproben, Umweltproben und biologischen Proben ausgewählt ist, wobei das Verfahren den Schritt des gleichzeitigen Nachweises des Vorhandenseins und/oder der Menge von Bakterien von mindestens zwei oder mehr verschiedenen Gattungen in der Probe auf der Grundlage von Antigen-Antikörper-Reaktionen umfasst,
wobei der Nachweis-Schritt umfasst: In Kontakt bringen der Probe mit einem Antikörper, der Antigen-Antikörper-Reaktionen mit Komponenten hervorruft, die von den Bakterien zweier oder mehrerer Gattungen stammen; und Messen des Vorhandenseins und/oder der Intensität der Antigen-Antikörper-Reaktion, die in der Probe nach dem Kontakt auftritt,
wobei der Antikörper ein monoklonaler Antikörper ist mit:
als Sequenz der variablen Region der schweren Kette weist die Aminosäuresequenz die Aminosäuresequenz von SEQ ID NO:1 auf, und als Sequenz der variablen Region der leichten Kette weist die Aminosäuresequenz die Aminosäuresequenz von SEQ ID NO:2 auf; oder
als Sequenz der variablen Region der schweren Kette weist die Aminosäuresequenz die Aminosäuresequenz von SEQ ID NO:3 auf, und als Sequenz der variablen Region der leichten Kette weist die Aminosäuresequenz die Aminosäuresequenz von SEQ ID NO:4 auf; oder
als Sequenz der variablen Region der schweren Kette weist die Aminosäuresequenz die Aminosäuresequenz von SEQ ID NO:5 auf, und als Sequenz der variablen Region der leichten Kette weist die Aminosäuresequenz die Aminosäuresequenz von SEQ ID NO:6 auf.

2. Verfahren nach Anspruch 1, wobei der des Nachweis-Schritt den gleichzeitigen Nachweis von Bakterien von zwei oder mehr, oder 3 oder mehr, oder 4 oder mehr, oder 5 oder mehr, oder 6 oder mehr, oder 7 oder mehr, oder 8 oder mehr, oder 9 oder mehr, oder 10 oder mehr, oder 11 oder mehr verschiedene Gattungen, ausgewählt aus der Gruppe bestehend aus der Gattung Escherichia, der Gattung Staphylococcus, der Gattung Pseudomonas, der Gattung Bacillus, der Gattung Klebsiella, der Gattung Serratia, der Gattung Rahnella, der Gattung Citrobacter, der Gattung Listeria, der Gattung Enterobacter und der Gattung Salmonella, beinhaltet
wobei die nachzuweisenden Bakterien von zwei oder mehr Gattungen gegebenenfalls ein gramnegatives Bakterium und ein grampositives Bakterium umfassen.

3. Verfahren nach Anspruch 1 oder 2, wobei der Antikörper ein Antikörper ist, der Antigen-Antikörper-Reaktionen mit den Ribosomenproteinen L7/L12 der Bakterien der zwei oder mehr Gattungen hervorruft.

4. Verfahren nach Anspruch 3, wobei der Antikörper keine Kreuzreaktionen mit mindestens einer nicht-bakteriellen Komponente verursacht, die in der Probe vorhanden sein kann,
wobei es sich bei der nichtbakteriellen Komponente, mit der der Antikörper keine Kreuzreaktionen verursacht, gegebenenfalls um eine organische Komponente handelt, die von einem Virus, einer Pflanze und/oder einem Tier stammt.

5. Verfahren nach einem der Ansprüche 1 bis 4, umfassend den Nachweis des Vorhandenseins und/oder der Menge von Bakterien in der Probe durch die folgenden Schritte:
(I) Einfangen der Bakterien in der Probe und Nachweis der Bakterien in der Probe auf der Grundlage von Antigen-Antikörper-Reaktionen zwischen der Probe, einem an einen Festphasenträger gebundenen Einfang-Antikörper und einem Nachweis-Antikörper mit einer Nachweis-Markierung, und
(II) Nachweisen der in der Probe nachzuweisenden Bakterien auf der Grundlage der Nachweis-Markierung, wobei entweder der Einfang-Antikörper oder der Nachweis-Antikörper mindestens ein spezifischer Antikörper ist, der Antigen-Antikörper-Reaktionen mit einer oder mehreren nachzuweisenden Bakterien verursacht, und der andere mindestens ein generischer Antikörper ist, der Antigen-Antikörper-Reaktionen mit Bakterien von fünf oder mehr Gattungen einschließlich der nachzuweisenden Bakterien verursacht, und
wobei entweder der generische Antikörper oder der spezifische Antikörper ein Antikörper ist, wie er in einem der Ansprüche 1 bis 4 angegeben ist.

6. Verfahren nach Anspruch 5, wobei Schritt (I) die folgenden Schritte umfasst:
(Ia-1) In Kontakt bringen des Nachweis-Antikörpers mit der Probe, um dadurch Bakterien in der Probe auf der Grundlage der Antigen-Antikörper-Reaktion zwischen dem Nachweis-Antikörper und den Bakterien nachzuweisen; und
(Ia-2) In Kontakt bringen des Einfang-Antikörpers mit der Probe, die die durch den Nachweis-Antikörper markierten Bakterien enthält, um dadurch die Bakterien in der Probe auf der Grundlage der Antigen-Antikörper-Reaktion zwischen dem Einfang-Antikörper und dem Bakterien-Nachweis-Antikörper-Komplex einzufangen, oder
wobei Schritt (I) die folgenden Schritte umfasst:
(Ib-1) In Kontakt bringen des Einfang-Antikörpers mit der Probe, um dadurch Bakterien in der Probe auf der Grundlage der Antigen-Antikörper-Reaktion zwischen dem Einfang-Antikörper und den Bakterien einzufangen, und
(Ib-2) In Kontakt bringen des Nachweis-Antikörpers mit der Probe, die die von den Einfang-Antikörper eingefangenen Bakterien enthält, um dadurch die Bakterien in der Probe auf der Grundlage der Antigen-Antikörper-Reaktion zwischen dem Nachweis-Antikörper und dem Bakterien-Einfang-Antikörper-Komplex nachzuweisen.

7. Verfahren nach Anspruch 5 oder 6, wobei der Einfang-Antikörper der generische Antikörper ist und der Nachweis-Antikörper der spezifische Antikörper ist, oder
wobei der Nachweis-Antikörper der generische Antikörper ist und der Einfang-Antikörper der spezifische Antikörper ist.

8. Verfahren nach einem der Ansprüche 5 bis 7, wobei der generische Antikörper Antigen-Antikörper-Reaktionen mit Bakterien von fünf oder mehr Gattungen, ausgewählt aus mindestens der Gattung Escherichia, der Gattung Staphylococcus, der Gattung Pseudomonas, der Gattung Bacillus, der Gattung Klebsiella, der Gattung Serratia, der Gattung Rahnella, der Gattung Citrobacter, der Gattung Listeria, der Gattung Enterobacter und der Gattung Salmonella, hervorruft und/oder
wobei der spezifische Antikörper Antigen-Antikörper-Reaktionen spezifisch mit Bakterien einer oder mehrerer Gattungen, ausgewählt aus mindestens der Gattung Escherichia, der Gattung Staphylococcus, der Gattung Pseudomonas, der Gattung Bacillus, der Gattung Klebsiella, der Gattung Serratia, der Gattung Rahnella, der Gattung Citrobacter, der Gattung Listeria, der Gattung Enterobacter und der Gattung Salmonella, hervorruft.

9. Verfahren nach einem der Ansprüche 5 bis 8, wobei der generische Antikörper umfasst:
als Sequenz der variablen Region der schweren Kette weist die Aminosäuresequenz die Aminosäuresequenz von SEQ ID NO:1 auf, und als Sequenz der variablen Region der leichten Kette weist die Aminosäuresequenz die Aminosäuresequenz von SEQ ID NO:2 auf; oder
als Sequenz der variablen Region der schweren Kette weist die Aminosäuresequenz die Aminosäuresequenz von SEQ ID NO:3 auf, und als Sequenz der variablen Region der leichten Kette weist die Aminosäuresequenz mit die Aminosäuresequenz von SEQ ID NO:4 auf; oder
als Sequenz der variablen Region der schweren Kette weist die Aminosäuresequenz die Aminosäuresequenz von SEQ ID NO:5 auf, und als Sequenz der variablen Region der leichten Kette weist die Aminosäuresequenz die Aminosäuresequenz von SEQ ID NO:6 auf, und/oder
wobei der spezifische Antikörper umfasst:
als Sequenz der variablen Region der schweren Kette weist die Aminosäuresequenz die Aminosäuresequenz von SEQ ID NO:7 auf, und als Sequenz der variablen Region der leichten Kette weist die Aminosäuresequenz die Aminosäuresequenz von SEQ ID NO:8 auf; oder
als Sequenz der variablen Region der schweren Kette weist die Aminosäuresequenz die Aminosäuresequenz von SEQ ID NO:9, und als Sequenz der variablen Region der leichten Kette weist die Aminosäuresequenz die Aminosäuresequenz von SEQ ID NO:10 auf; oder
als Sequenz der variablen Region der schweren Kette weist die Aminosäuresequenz die Aminosäuresequenz von SEQ ID NO:11 auf, und als Sequenz der variablen Region der leichten Kette weist die Aminosäuresequenz die Aminosäuresequenz von SEQ ID NO:12 auf; oder
als Sequenz der variablen Region der schweren Kette weist die Aminosäuresequenz die Aminosäuresequenz von SEQ ID NO:13 auf, und als Sequenz der variablen Region der leichten Kette weist die Aminosäuresequenz die Aminosäuresequenz von SEQ ID NO:14 auf.

10. Verfahren zur Bestimmung des Grades der Kontamination durch Bakterien in einer Probe, die aus Lebensmittel- oder Getränkeproben, Umweltproben und biologischen Proben ausgewählt wird, umfassend den Schritt des gleichzeitigen Nachweises des Vorhandenseins und/oder der Menge von Bakterien einer oder zweier oder mehrerer Gattungen in der Probe auf der Grundlage von Antigen-Antikörper-Reaktionen durch das Verfahren nach einem der Ansprüche 1 bis 9.

11. Kit zum Nachweis des Vorhandenseins und/oder der Menge von Bakterien in einer Probe, ausgewählt aus Lebensmittel- oder Getränkeproben, Umweltproben und biologischen Proben, durch das Verfahren nach einem der Ansprüche 1 bis 4, umfassend einen Antikörper, wie er in einem der Ansprüche 1 und 3 bis 4 angegeben ist.

12. Kit zum Nachweis des Vorhandenseins und/oder der Menge von Bakterien in einer Probe, ausgewählt aus Lebensmittel- oder Getränkeproben, Umweltproben und biologischen Proben, durch das Verfahren nach einem der Ansprüche 5 bis 9, umfassend einen Einfang-Antikörper und einen Nachweis-Antikörper, wie in einem der Ansprüche 5 bis 9 angegeben, wobei entweder der Einfang-Antikörper oder der Nachweis-Antikörper der generische Antikörper und der andere der spezifische Antikörper ist,
gegebenenfalls umfasst der Kit ferner einen unlöslichen Membranträger zum Entwickeln der Probe und zum In Kontakt bringen der Probe mit dem Einfang-Antikörper umfasst, wobei der unlösliche Membranträger eine darauf gebildete Nachweislinie aufweist und der Einfang-Antikörper auf der Nachweislinie immobilisiert ist, wodurch zwei oder mehr Bakterienarten in der Probe auf der einzelnen Nachweislinie nachgewiesen werden.

13. Kit nach Anspruch 11 oder 12, bei dem es sich um ein Immunochromatographie-Kit handelt, wobei der Einfang-Antikörper und der Nachweis-Antikörper gegebenenfalls so ausgewählt sind, dass sie Antigen-Antikörper-Reaktionen mit dem Ribosomenprotein L7/L12 der in der Probe nachzuweisenden Bakterien unter Bildung einer Sandwich-Struktur verursachen,
wobei das Immunochromatographie-Kit ferner umfasst:
einen unlöslichen Membranträger zum Entwickeln der Probe und zum In Kontakt bringen der Probe mit dem Einfang-Antikörper; und
ein Konjugatpad, das auf dem unlöslichen Membranträger gebildet ist und an das der Nachweisantikörper gebunden ist, und
wobei der Einfang-Antikörper an dem Konjugat-Pad auf dem unlöslichen Membranträger entlang der Entwicklungsrichtung der Chromatographie immobilisiert ist.

14. Verfahren zur Herstellung eines Immunochromatographie-Kits nach Anspruch 13, umfassend die Schritte:
Anbringen des Konjugatkissens, an das der Nachweisantikörper gebunden ist, auf dem unlöslichen Membranträger; und
Immobilisieren des Einfang-Antikörpers an dem Konjugat-Pad auf dem unlöslichen Membranträger entlang der Entwicklungsrichtung der Chromatographie,
wobei gegebenenfalls der spezifische Antikörper als Einfang-Antikörper und der generische Antikörper als Nachweis-Antikörper verwendet wird.

## Revendications

1. Procédé de détection de la présence et/ou de la quantité de bactéries dans un échantillon choisi parmi les échantillons d'aliments ou de boissons, les échantillons environnementaux et les échantillons biologiques, le procédé comprenant l'étape de détection simultanée de la présence et/ou de la quantité d'au moins des bactéries de deux genres différents ou plus dans l'échantillon sur la base de réactions antigène-anticorps,
dans lequel l'étape de détection comprend: la mise en contact de l'échantillon avec un anticorps qui provoque des réactions antigène-anticorps avec des composants dérivés des bactéries de deux genres ou plus; et la mesure de la présence et/ou de l'intensité de la réaction antigène-anticorps qui se produit dans l'échantillon après la mise en contact,
dans lequel l'anticorps est un anticorps monoclonal ayant:
en tant que séquence de région variable de chaîne lourde, la séquence d'acides aminés ayant la séquence d'acides aminés de SEQ ID NO:1, et en tant que séquence de région variable de chaîne légère, la séquence d'acides aminés ayant la séquence d'acides aminés de SEQ ID NO:2; ou
en tant que séquence de région variable de chaîne lourde, la séquence d'acides aminés ayant la séquence d'acides aminés SEQ ID NO:3, et en tant que séquence de région variable de chaîne légère, la séquence d'acides aminés ayant la séquence d'acides aminés SEQ ID NO:4; ou
en tant que séquence de la région variable de la chaîne lourde, la séquence d'acides aminés ayant la séquence d'acides aminés SEQ ID NO:5, et en tant que séquence de la région variable de la chaîne légère, la séquence d'acides aminés ayant la séquence d'acides aminés SEQ ID NO:6.

2. Procédé selon la revendication 1, dans lequel l'étape de détection comprend la détection simultanée de bactéries de deux ou plus, ou 3 ou plus, ou 4 ou plus, ou 5 ou plus, ou 6 ou plus, ou 7 ou plus, ou 8 ou plus, ou 9 ou plus, ou 10 ou plus, ou 11 ou plus genres différents, choisis dans le groupe constitué par le genre Escherichia, le genre Staphylococcus, le genre Pseudomonas, le genre Bacillus, le genre Klebsiella, le genre Serratia, le genre Rahnella, le genre Citrobacter, le genre Listeria, le genre Enterobacter et le genre Salmonella,
éventuellement, dans lequel les bactéries de deux genres ou plus à détecter comprennent une bactérie à Gram négatif et une bactérie à Gram positif.

3. Procédé selon la revendication 1 ou 2, dans lequel l'anticorps est un anticorps qui provoque des réactions antigène-anticorps avec les protéines L7/L12 du ribosome des bactéries de deux genres ou plus.

4. Procédé selon la revendication 3, dans lequel l'anticorps ne provoque pas de réactions croisées avec au moins un composant non bactérien pouvant être présent dans l'échantillon,
éventuellement, dans lequel le composant non bactérien avec lequel l'anticorps ne provoque pas de réactions croisées est un composant organique dérivé d'un virus, d'une plante et/ou d'un animal.

5. Procédé selon l'une des revendications 1 à 4, comprenant la détection de la présence et/ou de la quantité de bactéries dans l'échantillon par les étapes suivantes:
(I) capturer les bactéries dans l'échantillon et détecter les bactéries dans l'échantillon sur la base de réactions antigène-anticorps entre l'échantillon, un anticorps de capture lié à un support en phase solide, et un anticorps de détection ayant une étiquette de détection, et
(II) détection des bactéries à détecter dans l'échantillon sur la base de l'étiquette de détection, l'un de l'anticorps de capture et de l'anticorps de détection étant au moins un anticorps spécifique, qui provoque des réactions antigène-anticorps avec une ou plusieurs bactéries à détecter, et l'autre étant au moins un anticorps générique, qui provoque des réactions antigène-anticorps avec des bactéries de cinq genres ou plus, y compris la bactérie à détecter, et
dans lequel l'anticorps générique ou l'anticorps spécifique est un anticorps tel que décrit dans l'une des revendications 1 à 4.

6. Procédé selon la revendication 5, dans lequel l'étape (I) comprend les étapes suivantes:
(Ia-1) mettre en contact l'anticorps de détection avec l'échantillon pour détecter les bactéries dans l'échantillon sur la base de la réaction antigène-anticorps entre l'anticorps de détection et les bactéries; et
(Ia-2) mettre en contact de l'anticorps de capture avec l'échantillon contenant les bactéries marquées par l'anticorps de détection afin de capturer les bactéries dans l'échantillon sur la base de la réaction antigène-anticorps entre l'anticorps de capture et le complexe bactérie-anticorps de détection, ou
dans lequel l'étape (I) comprend les étapes suivantes :
(Ib-1) mettre en contact l'anticorps de capture avec l'échantillon pour capturer les bactéries dans l'échantillon sur la base de la réaction antigène-anticorps entre l'anticorps de capture et les bactéries, et
(Ib-2) mettre en contact de l'anticorps de détection avec l'échantillon contenant les bactéries capturées par l'anticorps de capture afin de détecter les bactéries dans l'échantillon sur la base de la réaction antigène-anticorps entre l'anticorps de détection et le complexe bactérie-anticorps de capture.

7. Procédé selon la revendication 5 ou 6, dans lequel l'anticorps de capture est l'anticorps générique et l'anticorps de détection est l'anticorps spécifique, ou
dans lequel l'anticorps de détection est l'anticorps générique et l'anticorps de capture est l'anticorps spécifique.

8. Procédé selon l'une des revendications 5 à 7, dans lequel l'anticorps générique provoque des réactions antigène-anticorps avec des bactéries de cinq genres ou plus choisis parmi au moins le genre Escherichia, le genre Staphylococcus, le genre Pseudomonas, le genre Bacillus, le genre Klebsiella, le genre Serratia, le genre Rahnella, le genre Citrobacter, le genre Listeria, le genre Enterobacter, et le genre Salmonella, et/ou
dans lequel l'anticorps spécifique provoque des réactions antigène-anticorps spécifiquement avec des bactéries d'un ou plusieurs genres choisis parmi au moins le genre Escherichia, le genre Staphylococcus, le genre Pseudomonas, le genre Bacillus, le genre Klebsiella, le genre Serratia, le genre Rahnella, le genre Citrobacter, le genre Listeria, le genre Enterobacter et le genre Salmonella.

9. Procédé selon l'une des revendications 5 à 8, dans lequel l'anticorps générique comprend:
en tant que séquence de la région variable de la chaîne lourde, la séquence d'acides aminés ayant la séquence d'acides aminés SEQ ID NO:1, et en tant que séquence de la région variable de la chaîne légère, la séquence d'acides aminés ayant la séquence d'acides aminés SEQ ID NO:2; ou
en tant que séquence de région variable de chaîne lourde, la séquence d'acides aminés ayant la séquence d'acides aminés SEQ ID NO:3, et en tant que séquence de région variable de chaîne légère, la séquence d'acides aminés ayant la séquence d'acides aminés SEQ ID NO:4; ou
en tant que séquence de région variable de chaîne lourde, la séquence d'acides aminés ayant la séquence d'acides aminés SEQ ID NO:5, et en tant que séquence de région variable de chaîne légère, la séquence d'acides aminés ayant la séquence d'acides aminés SEQ ID NO:6, et/ou
dans lequel l'anticorps spécifique comprend:
en tant que séquence de la région variable de la chaîne lourde, la séquence d'acides aminés ayant la séquence d'acides aminés SEQ ID NO:7, et en tant que séquence de la région variable de la chaîne légère, la séquence d'acides aminés ayant la séquence d'acides aminés SEQ ID NO:8; ou
en tant que séquence de la région variable de la chaîne lourde, la séquence d'acides aminés ayant la séquence d'acides aminés SEQ ID NO:9, et en tant que séquence de la région variable de la chaîne légère, la séquence d'acides aminés ayant la séquence d'acides aminés SEQ ID NO:10; ou
en tant que séquence de région variable de chaîne lourde, la séquence d'acides aminés ayant la séquence d'acides aminés SEQ ID NO:11, et en tant que séquence de région variable de chaîne légère, la séquence d'acides aminés ayant la séquence d'acides aminés SEQ ID NO:12; ou
en tant que séquence de la région variable de la chaîne lourde, la séquence d'acides aminés ayant la séquence d'acides aminés SEQ ID NO:13, et en tant que séquence de la région variable de la chaîne légère, la séquence d'acides aminés ayant la séquence d'acides aminés SEQ ID NO:14.

10. Procédé pour déterminer le degré de contamination par des bactéries dans un échantillon choisi parmi les échantillons d'aliments ou de boissons, les échantillons environnementaux et les échantillons biologiques, comprenant l'étape de détection simultanée de la présence et/ou de la quantité de bactéries d'un ou de deux genres ou plus dans l'échantillon sur la base de réactions antigène-anticorps par le procédé selon l'une quelconque des revendications 1 à 9.

11. Kit pour détecter la présence et/ou la quantité de bactéries dans un échantillon choisi parmi les échantillons d'aliments ou de boissons, les échantillons environnementaux et les échantillons biologiques par le procédé selon l'une quelconque des revendications 1 à 4, comprenant un anticorps tel que décrit dans l'une quelconque des revendications 1 et 3 à 4.

12. Kit pour détecter la présence et/ou la quantité de bactéries dans un échantillon choisi parmi les échantillons d'aliments ou de boissons, les échantillons environnementaux et les échantillons biologiques par le procédé selon l'une des revendications 5 à 9, comprenant un anticorps de capture et un anticorps de détection tels que décrits dans l'une des revendications 5 à 9, où l'un de l'anticorps de capture et de l'anticorps de détection est l'anticorps générique et l'autre est l'anticorps spécifique,
éventuellement, le kit comprend en outre un support à membrane insoluble pour développer l'échantillon et mettre en contact l'échantillon avec l'anticorps de capture, dans lequel le support de membrane insoluble comporte une ligne de détection la dessus et l'anticorps de capture est immobilisé sur la ligne de détection, ce qui permet de détecter deux espèces de bactéries ou plus dans l'échantillon sur la seule ligne de détection.

13. Kit selon la revendication 11 ou 12, qui est un kit d'immunochromatographie, éventuellement dans lequel l'anticorps de capture et l'anticorps de détection sont sélectionnés de manière à provoquer des réactions antigène-anticorps avec la protéine ribosomique L7/L12 de la bactérie à détecter dans l'échantillon pour former une structure en sandwich,
dans lequel le kit d'immunochromatographie comprend en outre
un support à membrane insoluble pour développer l'échantillon et le mettre en contact avec l'anticorps de capture; et
un tampon conjugué formé sur le support de membrane insoluble et auquel l'anticorps de détection est attaché, et
dans lequel l'anticorps de capture est immobilisé sur le tampon conjugué sur le support de membrane insoluble le long de la direction de développement de la chromatographie.

14. Procédé de fabrication d'un kit d'immunochromatographie selon la revendication 13, comprenant les étapes suivantes:
placer, sur le support de membrane insoluble, le tampon conjugué auquel l'anticorps de détection est attaché; et
immobiliser l'anticorps de capture sur le tampon conjugué sur le support de membrane insoluble le long de la direction de développement de la chromatographie,
éventuellement, l'anticorps spécifique est utilisé comme anticorps de capture et l'anticorps générique est utilisé comme anticorps de détection.
